# EUROPEAN PATENT APPLICATION

(11) **EP 3 263 700 A1**
(43) Date of publication of application: **03.01.2018**
(21) Application number: 16177150.6
(22) Date of filing: 30.06.2016
(51) Int. Cl.: C12N 9/48, A61K 38/00, C12N 9/64

(54) **USES OF DIPEPTIDYL PEPTIDASE 7 (DPP7) POLYPEPTIDE**

(71) Applicant: Université de Namur, 5000 Namur (BE)
(72) Inventor: Hack, Katrin, 5100 Naninne (BE); Cornelis, Guy Richard, 5332 Crupet (BE); Renzi, Francesco, 5000 Namur (BE); Dogné, Jean-Michel Paul Nicolas, 4030 Grivegnée (BE); Jonathan, Douxfils, 5336 Courrière (BE)
(74) Representative: Bogaerts, Sven

(57) **Abstract**

The present invention is situated in the field of DPP7 peptides. More particularly, the invention provides uses of these peptides, kits comprising these peptides, methods of producing these peptides, nucleic acid constructs comprising the nucleic acid sequence encoding these peptides and recombinant expression vectors and recombinant host cells comprising these nucleic acid constructs.

<110>  Université de Namur

<120>  USES OF DIPEPTIDYL PEPTIDASE 7 (DPP7) POLYPEPTIDE

<130>  UNA-004-EP-PRIO

<160>  7

<170>  PatentIn version 3.5

<210>  1
<211>  714
<212>  PRT
<213>  C. canimorsus DPP7

<400>  1

Met Met Lys Lys Ile Phe Thr Leu Ile Met Thr Phe Phe Val Gly Leu
1               5                   10                  15

Pro Ile Met Arg Ala Asp Glu Gly Met Trp Phe Leu Met Phe Val Glu
            20                  25                  30

Arg Leu Asn Gln Arg Asp Met Gln Lys Met Gly Leu Gln Leu Thr Ala
        35                  40                  45

Gln Glu Ile Tyr Ser Ile Asn Lys Asn Ser Leu Lys Asp Ala Ile Val
    50                  55                  60

Gln Phe Asn Gly Gly Cys Thr Ala Ser Ile Ile Ser Arg Asp Gly Leu
65                  70                  75                  80

Val Leu Thr Asn His His Cys Gly Tyr Gly Gln Ile Ala Glu Val Ser
                85                  90                  95

Ser Pro Glu Asn Asn Tyr Leu Arg Asp Gly Phe Trp Ala Ala Thr His
            100                 105                 110

Lys Asp Glu Ile Lys Pro Gln Ser Leu Phe Val Arg Phe Phe Val Arg
        115                 120                 125

Met Asp Asn Val Thr Glu Arg Met Leu Gly Val Val Asn Asp Gln Met
    130                 135                 140

Thr Glu Lys Glu Arg Glu Ala Ala Leu Asn Arg Glu Met Ala Lys Ile
145                 150                 155                 160

Gln Lys Glu Asn Asp Gly Gly Gly Lys Tyr Val Val Ser Val Arg Pro
                165                 170                 175

Phe Phe Gln Gly Asn Glu Tyr Tyr Tyr Phe Val Tyr Gln Asp Phe Lys
            180                 185                 190

Asp Val Arg Leu Val Gly Thr Pro Thr Glu Ser Ile Gly Lys Phe Gly
        195                 200                 205

Gly Asp Thr Asp Asn Trp Glu Trp Pro Arg Gln Thr Gly Asp Phe Ser
    210                 215                 220

Leu Phe Arg Val Tyr Thr Asp Lys Asn Gly Asn Ala Ala Glu Tyr Ser
225                 230                 235                 240

Pro Glu Asn Ile Pro Leu Arg Thr Glu Asn Tyr Leu Lys Ile Asn Ile
                245                 250                 255

Gly Gly Val Lys Glu Asn Asp Phe Ala Met Ile Leu Gly Tyr Pro Gly
            260                 265                 270

Arg Thr Asn Arg Trp Val Pro Ser Phe Trp Val Asp Gln Asn Val Lys
        275                 280                 285

Tyr Ala Tyr Pro Ala Trp Val Glu Ala Ser Lys Thr Ala Met Asp Ala
    290                 295                 300

Met Lys Pro Phe Met Asp Ala Asp Pro Ala Ile Arg Leu Met Tyr Ala
305                 310                 315                 320

Ser Lys Tyr Ala Gln Ile Ala Asn Tyr Trp Lys Asn Arg Gln Gly Met
                325                 330                 335

Ile Glu Ala Leu Thr Ala His Lys Thr Ala Glu Thr Lys Arg Lys Tyr
            340                 345                 350

Glu Lys Lys Phe Ala Lys Trp Ala Asn Arg Lys Lys Asn Arg Asp Gln
        355                 360                 365

Tyr Ala Gln Val Leu Pro Thr Met Gln Asn Tyr Phe Glu Lys Thr Asn
    370                 375                 380

Gln Tyr Ser Ala Asp Arg Asn Tyr Leu Met Ser Leu Leu Arg Ala Thr
385                 390                 395                 400

Asn Phe Ala Thr Val Pro Asn Thr Ile Ala Leu Gln Leu Gln Arg Leu
                405                 410                 415

Glu Gln Leu Pro Lys Glu Gln Gln Thr Ala Leu Leu Glu Arg Phe Lys
            420                 425                 430

Asn Leu Ser Ala Ile Tyr Asp Glu Met His Ile Pro Thr Glu Lys Ala
        435                 440                 445

Ile Leu Thr Asn Gly Leu Thr Leu Tyr Ala Ser Lys Ala Ala Asn Val
    450                 455                 460

Ser Gln Asp Val Lys Gln Ile Tyr Thr Asp Tyr Gln Gly Asp Phe Lys
465                 470                 475                 480

Lys Phe Val Glu Glu Ala Phe Gly Lys Ser Ile Phe Ala Ser Lys Glu
                485                 490                 495

Lys Tyr Ile Gln Phe Leu Glu Asn Pro Asp Ala Lys Ile Leu Glu Asn
            500                 505                 510

Asp Pro Leu Thr Gln Leu Ser Lys Ala Leu Met Thr Ala Tyr Asn Gln
        515                 520                 525

Lys Ser Glu Ser Ile Thr Ser Leu Glu Asp Gly Tyr Gln Lys Ala Tyr
    530                 535                 540

Arg Lys Phe Val Gln Gly Met Arg Lys Ser Gly Val Ser Arg Ile Gln
545                 550                 555                 560

Tyr Pro Asp Ala Asn Ser Thr Leu Arg Leu Thr Tyr Gly Thr Val Lys
                565                 570                 575

Ala Leu Pro Asn Asp Glu Arg Asn Ala Asp Ala Ser Ile Asn Asn Tyr
            580                 585                 590

Thr Thr Phe Lys Ser Met Val Ala Lys Tyr Lys Pro Asn Asp Pro Glu
        595                 600                 605

Phe Asp Met Pro Lys Arg Met Val Glu Met Tyr Glu Lys Asn Asp Phe
    610                 615                 620

Gly Arg Tyr Leu Asp Lys Asp Gly Thr Met His Val Asn Phe Leu Thr
625                 630                 635                 640

Asn Asn Asp Ile Thr Gly Gly Asn Ser Gly Ser Pro Val Met Asn Gly
                645                 650                 655

Lys Gly Glu Leu Ile Gly Leu Ala Phe Asp Gly Asn Ile Glu Ala Met
            660                 665                 670

Ala Gly Asp Val Ile Phe Asp Ala Lys Leu Gln Arg Thr Ile Asn Val
        675                 680                 685

Asp Ile Arg Tyr Val Leu Trp Val Ile Asp Lys Phe Ser Gly Ala Lys
    690                 695                 700

His Ile Val Asp Glu Met Thr Leu Val Glu
705                 710

<210>  2
<211>  2145
<212>  DNA
<213>  C. canimorsus DPP7

<400>  2
atgatgaaaa agatttttac actcattatg acgttctttg ttggattacc aataatgcgt      60

gccgacgaag gaatgtggtt tttgatgttt gttgagcgtc tcaatcagcg agatatgcaa     120

aaaatgggac ttcaactcac tgcccaagaa atttacagta tcaataaaaa cagtttaaaa     180

gatgccatcg tgcaatttaa tggtggttgt acagcaagta tcatctccag agatggtttg     240

gtacttacca atcaccactg cggttatggg caaattgcgg aggtatcttc ccccgaaaat     300

aactacctac gtgacggatt ttgggctgca acgcataaag acgaaatcaa acctcagtcg     360

ctttttgtgc gtttctttgt gcgtatggat aatgtaacgg agcgtatgct tggtgtggta     420

aacgaccaaa tgacagaaaa agaacgcgaa gctgcactaa acagagaaat ggcgaaaatt     480

caaaaagaga atgacggagg tggaaaatac gtagtttccg tgcgtccttt cttccaaggc     540

aatgaatatt attactttgt ttatcaagat tttaaagatg ttcgattggt aggtacccca     600

accgaaagca tcgggaaatt cggtggagat actgacaatt gggaatggcc tcgccaaact     660

ggtgattttt ctctattccg tgtatatacc gacaaaaacg gaaatgcagc agaatattca     720

cctgaaaaca ttcctttaag gactgaaaat tatttaaaaa tcaacatcgg aggagttaaa     780

gaaaatgatt ttgcaatgat tttgggctat cctgggcgta ctaaccgttg ggttccttcg     840

ttttgggtgg atcaaaatgt aaaatatgct tatccggctt gggttgaagc ctcaaaaaca     900

gcaatggatg ctatgaaacc gtttatggat gccgaccctg ccatccgctt aatgtatgct     960

tctaaatatg cacaaattgc caattattgg aaaaacagac aaggtatgat agaggcactc    1020

acagcacata aaaccgccga aaccaaacgc aaatacgaga aaaagtttgc taagtgggcc    1080

aatagaaaga aaaatcgtga ccaatatgca caggttttac ccactatgca gaattatttt    1140

gagaaaacga atcaatattc ggcagaccga aattatctga tgtctctatt acgtgctact    1200

aattttgcta ctgtacccaa taccattgct ttacaattgc aacgcttaga gcaactcccc    1260

aaagagcaac aaacagccct acttgaacgc ttcaaaaacc tcagtgctat ttatgacgaa    1320

atgcatatcc ctacggaaaa agccatttta acaaatggac ttacattgta tgcatcaaaa    1380

gcagcaaatg tatcgcagga tgtaaaacaa atttacactg attatcaagg tgatttcaaa    1440

aaatttgtag aagaagcctt tggaaaaagt atttttgctt ccaaagaaaa gtatatccaa 1500

tttttggaaa atcctgatgc aaaaatattg gaaaacgacc cgcttacaca actttctaag 1560

gcattaatga ctgcatacaa ccagaaatca gaaagcatta cttcacttga agatggctat 1620

caaaaagcat atcgtaaatt tgttcaagga atgcgtaaat ctggggtaag tcgtattcaa 1680

taccccgatg ccaactctac attacgattg acttacggta cagttaaagc cctgcccaat 1740

gacgaacgta atgcagatgc aagcattaac aactatacta ctttcaagtc aatggttgcc 1800

aaatataaac ctaatgaccc cgaattcgat atgcctaaac gtatggtcga gatgtacgaa 1860

aaaaatgatt ttggaagata tttggacaaa gacggaacaa tgcacgtaaa cttcctgacc 1920

aataacgata ttacgggcgg aaattcaggt tcgccggtaa tgaatggaaa aggagaactt 1980

atcggtttgg cttttgatgg taacattgaa gcgatggctg gagacgttat ttttgatgca 2040

aaactacaac gtaccatcaa cgtagatatt cgttatgtgc tttgggtaat tgataaattt 2100

tctggagcca aacatattgt tgatgaaatg actttggtag aatag 2145

<210> 3
<211> 54
<212> PRT
<213> Artificial sequence

<220>
<223> Fragment

<400> 3

Ala Ile Val Gln Phe Asn Gly Gly Cys Thr Ala Ser Ile Ile Ser Arg
1               5                   10                  15

Asp Gly Leu Val Leu Thr Asn His His Cys Gly Tyr Gly Gln Ile Ala
            20                  25                  30

Glu Val Ser Ser Pro Glu Asn Asn Tyr Leu Arg Asp Gly Phe Trp Ala
        35                  40                  45

Ala Thr His Lys Asp Glu
    50

<210> 4
<211> 84
<212> PRT
<213> Artificial sequence

<220>
<223> Fragment

<400> 4

Asp Val Arg Leu Val Gly Thr Pro Thr Glu Ser Ile Gly Lys Phe Gly

1                   5                   10                  15

Gly Asp Thr Asp Asn Trp Glu Trp Pro Arg Gln Thr Gly Asp Phe Ser
            20                  25                  30

Leu Phe Arg Val Tyr Thr Asp Lys Asn Gly Asn Ala Ala Glu Tyr Ser
        35                  40                  45

Pro Glu Asn Ile Pro Leu Arg Thr Glu Asn Tyr Leu Lys Ile Asn Ile
    50                  55                  60

Gly Gly Val Lys Glu Asn Asp Phe Ala Met Ile Leu Gly Tyr Pro Gly
65                  70                  75                  80

Arg Thr Asn Arg

<210>  5
<211>  133
<212>  PRT
<213>  Artificial sequence

<220>
<223>  Fragment

<400>  5

Pro Asp Ala Asn Ser Thr Leu Arg Leu Thr Tyr Gly Thr Val Lys Ala
1                   5                   10                  15

Leu Pro Asn Asp Glu Arg Asn Ala Asp Ala Ser Ile Asn Asn Tyr Thr
            20                  25                  30

Thr Phe Lys Ser Met Val Ala Lys Tyr Lys Pro Asn Asp Pro Glu Phe
        35                  40                  45

Asp Met Pro Lys Arg Met Val Glu Met Tyr Glu Lys Asn Asp Phe Gly
    50                  55                  60

Arg Tyr Leu Asp Lys Asp Gly Thr Met His Val Asn Phe Leu Thr Asn
65                  70                  75                  80

Asn Asp Ile Thr Gly Gly Asn Ser Gly Ser Pro Val Met Asn Gly Lys
                85                  90                  95

Gly Glu Leu Ile Gly Leu Ala Phe Asp Gly Asn Ile Glu Ala Met Ala
            100                 105                 110

Gly Asp Val Ile Phe Asp Ala Lys Leu Gln Arg Thr Ile Asn Val Asp
        115                 120                 125

Ile Arg Tyr Val Leu
    130



<210>  6
<211>  8
<212>  PRT
<213>  Artificial sequence

<220>
<223>  Fragment

<400>  6

Thr Asn His His Cys Gly Tyr Gly
1               5



<210>  7
<211>  12
<212>  PRT
<213>  Artificial sequence

<220>
<223>  Fragment

<400>  7

Asn Asn Asp Ile Thr Gly Gly Asn Ser Gly Ser Pro
1               5                   10

## Description

### TECHNICAL FIELD

The present invention is situated in the field of DPP7 peptides. More particularly, the invention provides uses of these peptides, kits comprising these peptides, methods of producing these peptides, nucleic acid constructs comprising the nucleic acid sequence encoding these peptides and recombinant expression vectors and recombinant host cells comprising these nucleic acid constructs.

### BACKGROUND OF THE INVENTION

Upon blood vessel injury, the loss of blood from the system must be stopped before shock and possible death occur. This is achieved by the process of blood coagulation or clotting which is a complex, multi-step process that requires many different coagulation factors. These complex reactions ensure that blood coagulation occurs rapidly and yet remains localized. Blood coagulation results in the formation of a plug of platelets enmeshed in a network of insoluble fibrin strands, called a haemostatic clot, which controls bleeding and serves as a nidus for subsequent cellular ingrowth and tissue repair. This process also involves a feedback system that regulates clot formation in the body so that clots are removed when the injury site is healed. Abnormalities that result in failure or delay of clot formation or clot removal are associated with an increased risk of bleeding.

Blood coagulation is typically regulated by two pathways involving numerous clotting factors: the initiation phase is triggered by the extrinsic pathway, whereas amplification requires the intrinsic pathway. The extrinsic pathway consists of the transmembrane receptor tissue factor (TF) and plasma factor VII/VIIa (FVII/FVIIa), and the intrinsic pathway consists of plasma factors FXI, FIX, and FVIII. In brief, damage to blood vessel walls exposes TF-containing cells from underlying cell layers to the blood. TF is then able to bind in the presence of calcium to FVII, which circulates at low levels in the bloodstream, the calcium forming a bridge between TF and FVII. This sets off an extracellular cascade involving sequential serine protease activations: TF/FVII is activated by auto-cleavage to TF/FVIIa, which along with FVIIIa (cofactor) converts FIX to FIXa, which converts FX to FXa (although TF/FVIIa can also directly convert FX to FXa), which along with FVa (cofactor) converts FII (prothrombin) to FIIa (thrombin), which converts fibrinogen to fibrin, leading to fibrin deposition and the activation of platelets to form blood clots. The activation of FXIII to FXIIIa stabilizes the fibrin clot by cross-linking the fibrin polymers.

Substances which disturb the process of blood coagulation, namely anticoagulants, have been demonstrated to be important therapeutic agents in the treatment and prevention of thrombotic disorders. Many anticoagulants, such as heparin and the coumarin derivatives warfarin or phenprocoumon have been associated with a number of adverse effects due to the relatively non-specific nature of their effects on the blood coagulation cascade. Besides, coumarin derivatives have a long plasma half-life, as well as a slow onset of action, while heparin has a short half-life and is a rapidly acting anticoagulant.

This has encouraged the search for more effective anticoagulant agents which can better control the activity of the coagulation cascade by selectively interfering with specific reactions in this process which may have a positive effect in reducing the complications of anticoagulant therapy, and which are fast-acting and could therefore be used in emergency situations.

### SUMMARY

The present invention provides uses of DPP7 peptides, or functionally active variants or fragments thereof, kits comprising these peptides, methods of producing these peptides, nucleic acid constructs comprising the nucleic acid sequence encoding these peptides and recombinant expression vectors and recombinant host cells comprising these nucleic acid constructs. Indeed, the inventors have discovered that DPP7 peptides, or functionally active variants or fragments thereof, can be used to cleave and inactivate coagulation factor X, VII and/or FIX. Accordingly, the DPP7 peptide, or functionally active variants or fragments thereof can be used as a fast- and long-acting anticoagulant, for producing FX-, FVII- and/or FIX-deficient plasma and for the production of an antidote against a FXa, FVIIa or FIXa inhibitor.

One aspect of the invention relates to a DPP7 peptide, or functionally active variants or fragments thereof, for use in the cleavage and inactivation of coagulation factor X, VII and/or IX. In particular embodiments, the DPP7 peptide, or functionally active variants or fragments thereof according to the invention are used in the treatment and/or prevention of a medical disorder chosen from the list comprising atrial fibrillation induced thromboembolism, pulmonary embolism, deep vein thrombosis, venous thromboembolism, congestive heart failure, stroke, coronary syndrome, peripheral arterial occlusion, myocardial infarction and/or genetic or acquired hypercoagulability. In particular embodiments, the DPP7 peptide, or functionally active variants or fragments thereof, is used as an anticoagulant. In particular embodiments, the DPP7 peptide, or functionally active variants or fragments thereof, is bacterial DPP7, preferably *Porphyromonas gingivalis* DPP7, *Capnocytophaga canimorsus* DPP7, *Capnocytophaga canis nov. sp.* DPP7 or *Capnocytophaga cynodegmi* DPP7, more preferably *Capnocytophaga canimorsus* DPP7. In particular embodiments, the DPP7 peptide comprises an amino acid sequence which has at least 90%, preferably at least 95%, most preferably 100% identity with the amino acid sequence set forth in SEQ ID NO:1, or functionally active variants or fragments thereof. In particular embodiments, the DPP7 peptide comprises:
(a) an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(b) an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(c) an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1 ; or functionally active variants or fragments thereof.

Another aspect of the invention relates to the *in vitro* use of a DPP7 peptide, or functionally active variants or fragments thereof, in the cleavage and inactivation of coagulation factor X, VII and/or IX. In particular embodiments, the DPP7 peptide, or functionally active variants or fragments thereof, for *in vitro* use according to present invention is further used in the production of an anticoagulant reversal agent, wherein said anticoagulant is a factor X(a), VII(a) or IX(a) inhibitor. In particular embodiments, the DPP7 peptide, or functionally active variants or fragments thereof, for *in vitro* use according to present invention is further used for depleting a plasma sample of coagulation factor X, VII and/or IX. In particular embodiments, the DPP7 peptide or functionally active variants or fragments thereof, is used in medical devices. In particular embodiments, the DPP7 peptide, or functionally active variants or fragments thereof, is used as an anticoagulant. In particular embodiments, the DPP7 peptide, or functionally active variants or fragments thereof, is bacterial DPP7, preferably *Porphyromonas gingivalis* DPP7, *Capnocytophaga canimorsus* DPP7, *Capnocytophaga canis nov. sp.* DPP7 or *Capnocytophaga cynodegmi* DPP7, more preferably *Capnocytophaga canimorsus* DPP7. In particular embodiments, the DPP7 peptide comprises an amino acid sequence which has at least 90%, preferably at least 95%, most preferably 100% identity with the amino acid sequence set forth in SEQ ID NO:1, or functionally active variants or fragments thereof. In particular embodiments, the DPP7 peptide comprises:
(a) an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(b) an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(c) an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1 ;
or functionally active variants or fragments thereof.

A further aspect of the invention relates to a kit comprising a DPP7 peptide, or functionally active variants or fragments thereof, wherein said peptide or functionally active variants or fragments thereof, has anticoagulant activity, preferably by inhibiting clotting factor X, VII and/or IX, preferably wherein said DPP7 peptide comprises an amino acid sequence which has at least 90%, preferably at least 95%, most preferably 100% identity with the amino acid sequence set forth in SEQ ID NO:1, or functionally active variants or fragments thereof.

A further aspect of the invention relates to a kit comprising one or more plasma samples depleted of coagulation factor X, VII and/or IX, wherein said one or more plasma samples are depleted of coagulation factor X, VII and/or IX using a DPP7 peptide or functionally active variants or fragments thereof.

A further aspect of the invention relates to a nucleic acid construct comprising a nucleic acid sequence which has
(a) at least 90%, preferably at least 95%, most preferably 100% identity with the nucleic acid sequence set forth in SEQ ID NO:2,
and/or encodes a peptide comprising:
(b) an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(c) an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(d) an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1,
operably linked to one or more control sequences capable of directing the expression of the polypeptide in a suitable expression host.

A further aspect of the invention relates to a recombinant expression vector comprising the nucleic acid construct according to the invention, a promoter, and transcriptional, translational stop signals, and preferably, a selectable marker.

A further aspect of the invention relates to a recombinant host cell comprising the nucleic acid construct according to the invention, preferably contained on a vector or integrated into the host cell genome. In particular embodiments, the host cell according to the invention is a bacterial cell, preferably *Escherichia coli, Porphyromonas gingivalis, Capnocytophaga canimorsus, Capnocytophaga canis* or *Capnocytophaga cynodegmi, Flavobacterium johnsoniae,* more preferably *Capnocytophaga canimorsus.*

A further aspect of the invention relates to a method for producing a polypeptide having protease activity comprising (a) cultivating the host cell according to the invention under conditions conducive to expression of the polypeptide; and (b) recovering the polypeptide.

### BRIEF DESCRIPTION OF THE FIGURES

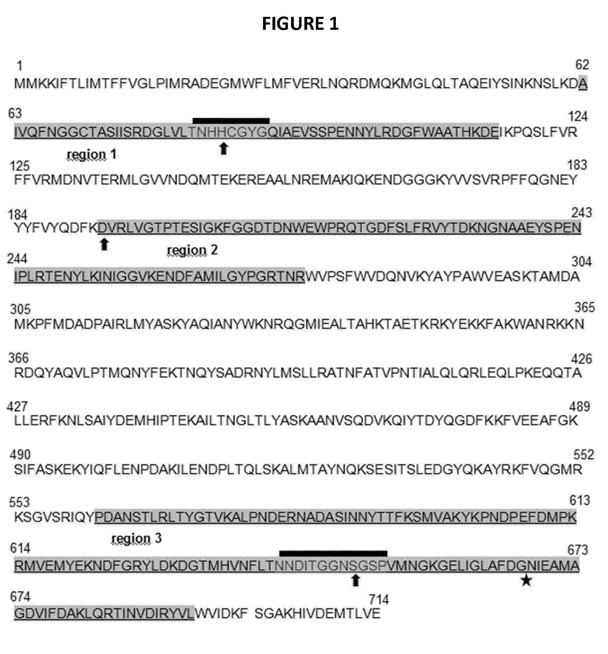

**Figure 1** shows the *C. canimorsus* DDP7 amino acid sequence (SEQ ID NO :1) with an indication of the three conserved regions (grey). These three conserved regions comprise subregions (indicated by a thick black line above the amino acid sequence) with highly similar amino acids residues. The essential amino acids that form the catalytic triad of the serine protease family, namely H86, D193 and S649, are indicated with an arrow. The amino acid indicated with a star, namely G667, is critical to define substrate specificity of C. *canimorsus* DPP7. The numbers above the amino acid sequence indicate the location of the amino acid sequence.

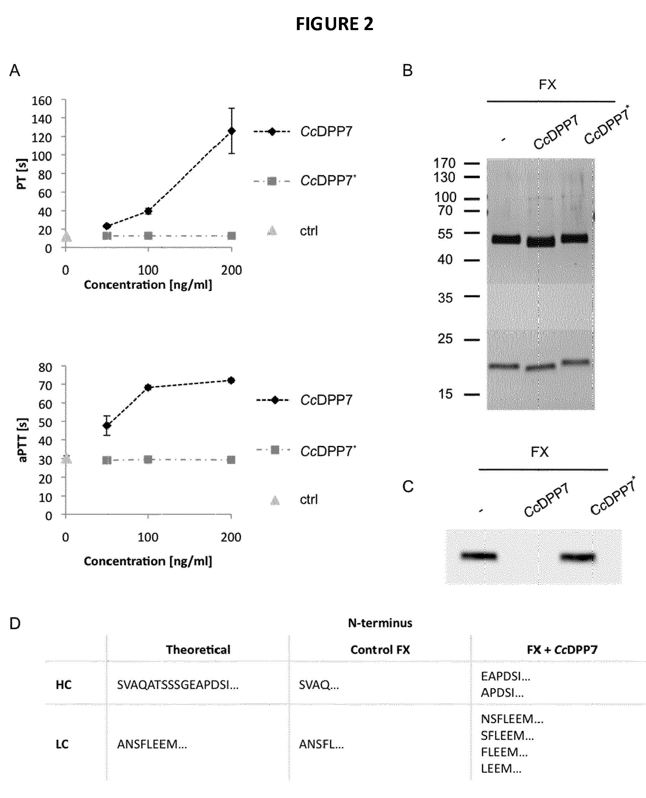

**Figure 2** shows cleavage and inactivation of FX by *C. canimorsus* DPP7. (A) *C. canimorsus* DPP7, the catalytic mutant *C. canimorsus* DPP7* or PBS were diluted 1:10 in NPP to the indicated concentrations and incubated at 37 °C for 1 h. (B+C) 50 µg/ml of purified human FX in PBS was incubated for 30 min in the presence or absence of 12.5 µg/ml of C. *canimorsus DPP7* or C. canimorsus DPP7 S649A. FX was visualized by silver staining. (B) Silver staining of FX incubated with *C. canimorsus* DPP7, DPP7* or PBS. (C). The N-terminus of FX LC was detected by WB with an antibody directed to aa 1-20. (D) 50 µg/ml of purified human FX in PBS was incubated for 30 min in the presence or absence of 12.5 µg/ml of *C. canimorsus DPP7.* Automated N-terminal Edman degradation was performed on LC and HC. Amino acid sequences from two independent experiments are shown. DPP7* is a catalytic inactive mutant of DPP7 where serine 649 was replaced by an alanine.

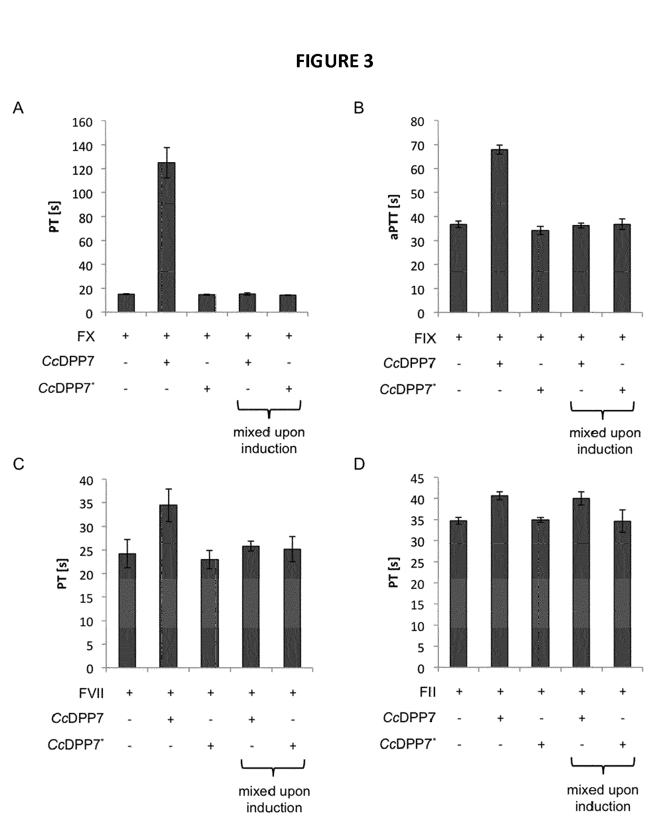

**Figure 3** shows FX, FIX, FVII, FII activity in factor depleted plasma after incubation with *C*. *canimorsus* DPP7, *C. canimorsus* DPP7* or PBS. 50 µg/ml of clotting factors were incubated in the presence or absence of 12.5 µg/ml of *C. canimorsus* DPP7 or *C*. *canimorsus* DPP7* for 30 min. FX and FIX were diluted 1:5 with plasma to their physiological plasma concentrations. FVII and FII were diluted 1:1 with plasma to 0.5 µg/ml or 10 µg/ml respectively to allow a more sensitive detection of functional impairment. To exclude interaction of *C. canimorsus* DPP7 with factor deficient plasma at the point of induction a control was included in which the factors and the protease were incubated separately and only mixed upon induction. DPP7* is a catalytic inactive mutant of DPP7 where serine 649 was replaced by an alanine.

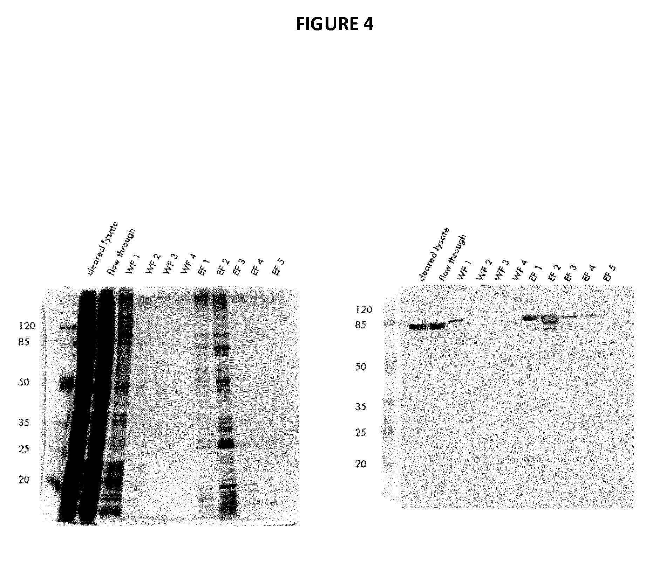

**Figure 4** shows a silver staining (left) and a western blot using an anti-his tag antibody (6-His monoclonal antibody, Eurogentec) (right) after the his-tag affinity chromatography for the purification of *C. canimorsus* DPP7 from *E. coli* BL21. The numbers on the left sides of the blots indicate the size of the protein in kDa.

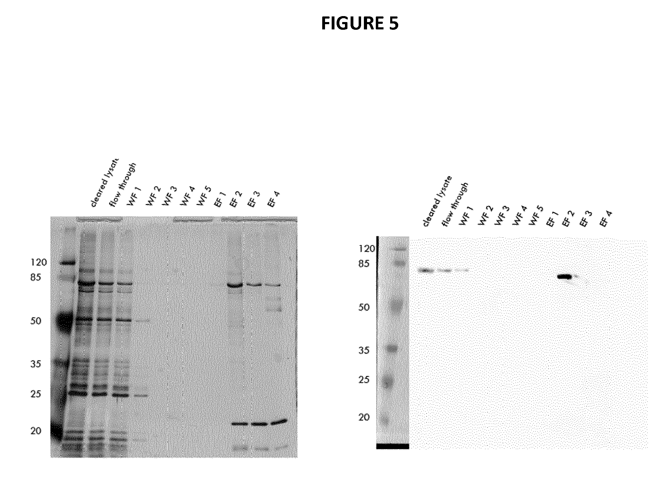

**Figure 5** shows a silver staining (left) and a western blot using an anti-strep tag antibody (anti-Strep tag classic HRP, Bio-Rad) (right) after the strep-tag affinity chromatography for the purification of *C. canimorsus* DPP7 from *E. coli* BL21. The numbers on the left sides of the blots indicate the size of the protein in kDa.

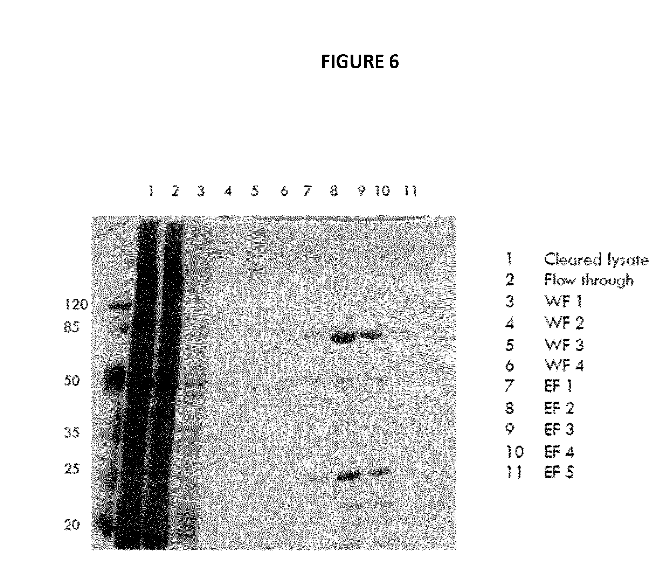

**Figure 6** shows a silver staining after the his-tag affinity chromatography for the purification of *C. canimorsus* DPP7 from *C. canimorsus* 5. The numbers on the left sides of the blots indicate the size of the protein in kDa.

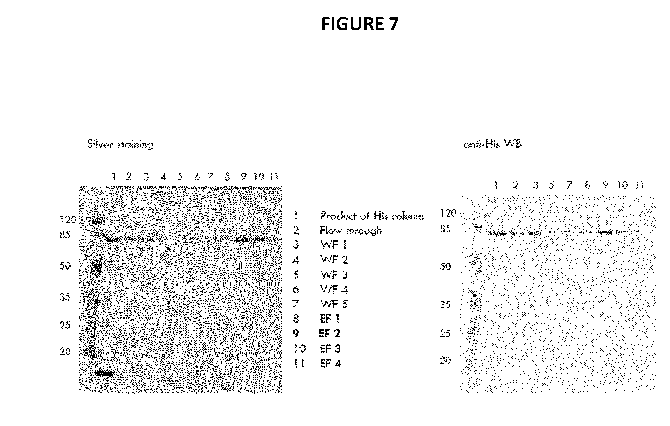

**Figure 7** shows the silver staining (left) and a western blot using an anti-his tag antibody (anti-Strep tag classic HRP, Bio-Rad) (right) after the strep-tag affinity chromatography for the purification of *C. canimorsus* DPP7 from *C. canimorsus* 5. The numbers on the left sides of the images indicate the size of the protein in kDa.

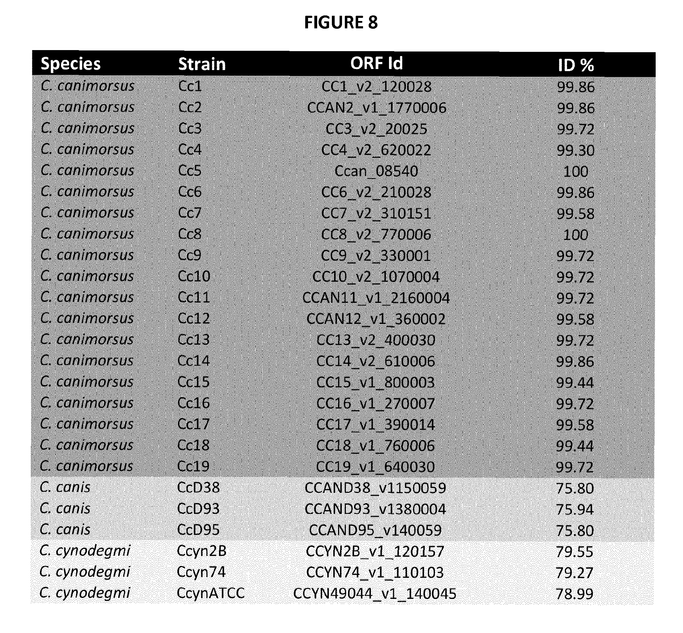

**Figure 8** shows homologs of *C. canimorsus 5 dppS* in dog hosted *Capnocytophaga* species. Shown is the percentage of gene identity with *dppS.* Homologs of *dppS* have been identified using the MicroScope Comparative genomics Gene Phyloprofile tool (https://www.genoscope.cns.fr/aqc/microscope/compgenomics/phyloprofil.php?) searching for homologs in all these sequenced strains of the dog hosted *Capnocytophaga* species *(C.canimorsus, C.canis, C.cynodegmi)* choosing as homology constraints parameters: min Lrap≥0.8; max Lrap≥0; Identity≥30%.

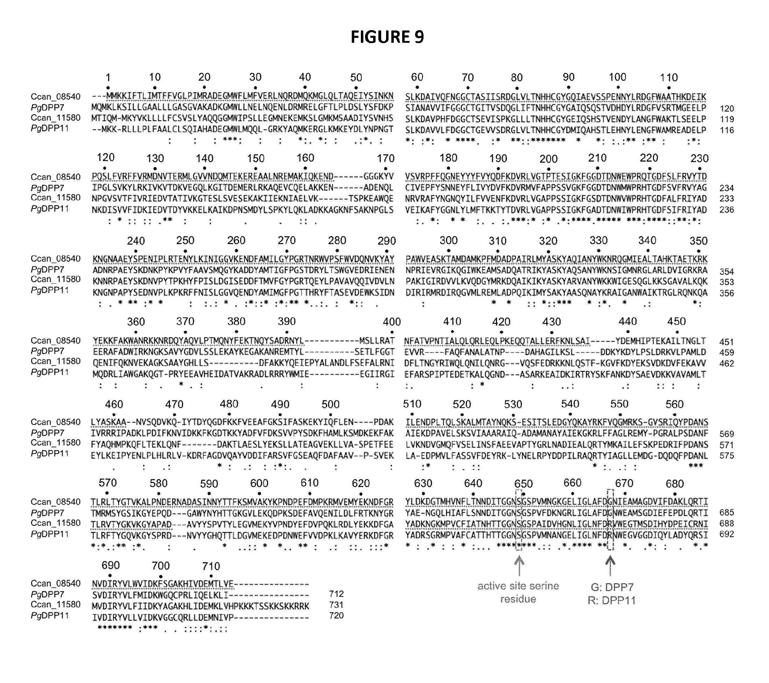

**Figure 9** shows the alignment of *Porphyromonas gingivalis* DPP7 and DPP11 with C. *Canimorsus* 5 DPP7 and DPP11. Protein sequences were aligned with the CLUSTAL Omega (http://www.ebi.ac.uk/Tools/msa/clustalo/). Numbers on the top indicate the aa position in Ccan_08540, numbers on the right indicate positions for Ccan_11580, P. *gingivalis* DPP7 and *P. gingivalis* DPP11. Asterisks (*) indicate full conservation of single residues, colons (:) indicate conservation between groups of strongly similar proteins and periods (.) indicate conservation between groups of weakly similar proteins.

*Abbreviations used:* HC, heavy chain; LC, light chain; Cc, *C. Canimorsus;* DPP7, Dipeptidyl-Peptidase 7; F, factor; WF, wash fraction; EF, elution fraction; PT, prothrombin time; S, seconds.

### DETAILED DESCRIPTION OF THE INVENTION

Before the present uses of these peptides, kits comprising these peptides, methods of producing these peptides, nucleic acid constructs comprising the nucleic acid sequence encoding these peptides and recombinant expression vectors and recombinant host cells comprising these nucleic acid constructs used in the invention are described, it is to be understood that this invention is not limited to particular uses, kits, methods, nucleic acid constructs, vectors and host cells described, as such particular uses, kits, methods, nucleic acid constructs, vectors and host cells may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

*Capnocytophaga canimorsus,* a Gram-negative bacterium belonging to the oral flora of dogs, causes severe sepsis along with bleeding abnormalities in humans that have been bitten, licked or scratched. The Applicants surprisingly discovered that a new peptidase which they isolated from *Capnocytophaga canimorsus* and assigned as a dipeptidyl peptidase 7 (DPP7) type protease of the S46 serine protease family (Figure 9), cleaves FX, FVII and FIX. For example, DPP7 degrades FX heavy and light chain from the N-terminus and renders FX dysfunctional, thereby contributing to inhibit coagulation.

Accordingly, the present invention is based in part on the identification of amino acid sequences of bacterial protease peptides that are related to the DPP7 subtype of the S46 protease family, as well as functionally active variants, fragments and mammalian orthologues thereof. These peptide sequences, as well as functionally active variants or fragments thereof, can be used to cleave and inactivate coagulation FX, FVII and/or FIX, preferably FX, and therefore could serve as an inhibitor of coagulation for *in vitro* and *in vivo* applications. Furthermore, these peptide sequences, as well as functionally active variants or fragments thereof, could be used to obtain FX-, FVII-, and/or FIX-deficient plasma and for obtaining an antidote against anticoagulants that specifically target FX(a), FVII(a) and/or FIX(a).

A first aspect of the invention provides a DPP7 peptide, or functionally active variants or fragments thereof, for use in the cleavage and inactivation of coagulation factor X, VII and/or IX, preferably factor X.

The terms "peptide", "polypeptide", or "protein" can be used interchangeably and relate to any natural, synthetic, or recombinant molecule comprising amino acids joined together by peptide bonds between adjacent amino acid residues. A "peptide bond", "peptide link" or "amide bond" is a covalent bond formed between two amino acids when the carboxyl group of one amino acid reacts with the amino group of the other amino acid, thereby releasing a molecule of water. The polypeptide can be from any source, e.g., a naturally occurring polypeptide, a chemically synthesized polypeptide, a polypeptide produced by recombinant molecular genetic techniques, or a polypeptide from a cell or translation system. Preferably, the polypeptide is a polypeptide produced by recombinant molecular genetic techniques. The polypeptide may be a linear chain or may be folded into a globular form. The terms "amino acid" and "amino acid residue" may be used interchangeably herein.

Prokaryote DPP7 type proteases belong to the S46 family of serine proteases. A member of this group, which has been characterized most extensively, is *Porphyromonas gingivalis* DPP7. The bacterium is dependent on extracellular peptides as a nutrient source and therefore expresses a vast array of proteases with different functions. *P. gingivalis* can only import dipeptides and is thus dependent on the synthesis of dipeptidases which further break-down oligopeptides. *P. gingivalis* DPP7 is anchored in the outer membrane which is perfectly in line with its function to degrade extracellular proteins. However, not all bacterial DPP7 type proteases have predicted signal peptides, for instance *C. canimorsus* DPP7, and are hence rather cytoplasmic. Their function is probably the recycling and degradation of intracellular proteins. Just now, the Applicants found that *C. canimorsus* DPP7 can degrade and inactivate FX, FVII and/or FIX, preferably FX, thereby mediating an inhibiting effect on coagulation.

The terms "Dipeptidyl-Peptidase 7", "dipeptidylpeptidase 7", "Dipeptidyl Peptidase 7" and "DPP7" can be used interchangeably herein.

DPP7 homologues can be found in many species. The DPP7 peptide according to present invention is preferably a bacterial DPP7.

The kingdom of Bacteria can be divided into several phyla such as *Bacteroidetes.* The phylum of *Bacteroidetes* can be further divided into several classes such as *Bacteroidia, Cytophagia, Flavobacteriia, Sphingobacteriia* and *Bacteroidetes incertai sedis.* The class of *Flavobacteriia* can be further divided into orders, such as the Flavobacteriales which are subdivided into genera, for example, *Capnocytophaga, Ornithobacterium* and *Coenonia.* The genus of *Capnocytophaga* can be further divided into species, such as *C. canimorsus, C. canis nov. sp., C. cynodegmi, C. gingivalis, C. granulosa, C. haemolytica, C. ochracea* and *C. sputigena.* These scientific classifications are known by the skilled person.

In particular embodiments, the DPP7 peptide, or functionally active variants or fragments thereof is a bacterial DPP7. Bacterial species may be all bacterial species known by the one skilled in the art. In more particular embodiments, the DPP7 peptide, or functionally active variants or fragments thereof is a member of the *Bacteroidetes* phylum DPP7.

In more particular embodiments, the DPP7 peptide, or functionally active variants or fragments thereof, for use according the invention is *Porphyromonas gingivalis* DPP7, *Capnocytophaga canimorsus* DPP7, *Capnocytophaga canis nov. sp.* DPP7 or *Capnocytophaga cynodegmi* DPP7, preferably *Capnocytophaga canimorsus* DPP7.

The genomes of 19 clinical isolates of *C. canimorsus,* collected by the inventors, have been sequenced and all of them possess the *C. canimorsus* DDP7 encoding nucleic acid sequence as set forth in SEQ ID NO:2 with almost 100% gene identity (Figure 8). More particularly, these *C. canimorsus* isolates are (in the inventors collection) *C. canimorsus* 2, *C. canimorsus* 3, *C. canimorsus* 5, *C. canimorsus* 6, *C. canimorsus* 7, *C. canimorsus* 8, *C. canimorsus* 9, *C. canimorsus* 10, *C. canimorsus* 11, *C. canimorsus* 12, *C. canimorsus* 13, C. *canimorsus* 14, *C. canimorsus* 15, *C. canimorsus* d6, *C. canimorsus* d43, *C. canimorsus* d68, *C. canimorsus* d38, *C. canimorsus* d 93 and *C. canimorsus* d95 , all isolated from the mouth of a dog, by the inventors, also possess the *C. canimorsus* DDP7 encoding nucleic acid sequence as set forth in SEQ ID NO:2 with almost 100% gene identity.

In further particular embodiments, the DPP7 peptide is a *C*. *canimorsus* 2 DPP7, *C*. *canimorsus* 3 DPP7, *C. canimorsus* 5 DPP7, *C. canimorsus* 6 DPP7, *C. canimorsus* 7 DPP7, *C. canimorsus* 8 DPP7, *C. canimorsus* 9 DPP7, *C. canimorsus* 10 DPP7, *C. canimorsus* 11 DPP7, *C. canimorsus* 12 DPP7, *C. canimorsus* 13 DPP7, *C. canimorsus* 14 DPP7, C. *canimorsus* 15 DPP7, *C. canimorsus* d6 DPP7, *C. canimorsus* d43 DPP7, *C. canimorsus* d68 DPP7, *C. canimorsus* d38 DPP7, *C. canimorsus* d 93 DPP7 or *C*. *canimorsus* d95 DPP7 peptide, preferably a *C. canimorsus 5* DPP7 peptide.

Exemplary *C*. *canimorsus* 5 DPP7 protein sequence may be as annotated under U.S. government's National Center for Biotechnology Information (NCBI) Genbank (http://www.ncbi.nlm.nih.gov/) accession number GI:754503141. *C. canimorsus 5* DPP7 (cDNA) sequence may be as annotated under NCBI Genbank accession number NC_015846.1.

The *C. canimorsus 5* DPP7 amino acid sequence annotated under GI:754503141 and corresponds with the amino acid sequence as set forth in SEQ ID NO :1.

**Table 1**

| **SEQ ID NO** | **TYPE** | **SEQUENCE** |
|---|---|---|
| SEQ ID NO:1 | amino acid | MMKKIFTLIMTFFVGLPIMRADEGMWFLMFVERLNQRDMQKMGLQLT AQEIYSINKNSLKDAIVQFNGGCTASIISRDGLVLTNHHCGYGGQIAEVSS PENNYLRDGFWAATHKDEIKPQSLFVRFFVRMDNVTERMLGVVNDQ MTEKEREAALNREMAKIQKENDGGGKYVVSVRPFFQGNEYYYFVYQ DFKDVRLVGTPTESIGKFGGDTDNWEWPRQTGDFSLFRVYTDKNGN AAEYSPENIPLRTENYLKINIGGVKENDFAMILGYPGRTNRWVPSFWV DQNVKYAYPAWVEASKTAMDAMKPFMDADPAIRLMYASKYAQIANY WKNRQGMIEALTAHKTAETKRKYEKKFAKWANRKKNRDQYAQVLPT MQNYFEKTNQYSADRNYLMSLLRATNFATVPNTIALQLQRLEQLPKEQ QTALLERFKNLSAIYDEMHIPTEKAILTNGLTLYASKAANVSQDVKQIYT DYQGDFKKFVEEAFGKSIFASKEKYIQFLENPDAKILENDPLTQLSKAL MTAYNQKSESITSLEDGYQKAYRKFVQGMRKSGVSRIQYPDANSTLR |
| | | LTYGTVKALPNDERNADASINNYTTFKSMVAKYKPNDPEFDMPKRMV EMYEKNDFGRYLDKDGTMHVNFLTNNDITGGNSGSPVMNGKGELIGL AFDGNIEAMAGDVIFDAKLQRTINVDIRYVLWVIDKF SGAKHIVDEMTLVE |
| SEQ ID NO:2 | nucleic acid | ATGATGAAAAAGATTTTACACTCATTATGACGTTCTTTGTTGGATTA CCAATAATGCGTGCCGACGAAGGAATGTGGTTTTTGATGTTTGTTG AGCGTCTCAATCAGCGAGATATGCAAAAAATGGGACTTCAACTCAC TGCCCAAGAAATTTACAGTATCAATAAAAACAGTTTAAAAGATGCCA TCGTGCAATTTAATGGTGGTTGTACAGCAAGTATCATCTCCAGAGA TGGTTTGGTACTTACCAATCACCACTGCGGTTATGGGCAAATTGCG GAGGTATCTTCCCCCGAAAATAACTACCTACGTGACGGATTTTGGG CTGCAACGCATAAAGACGAAATCAAACCTCAGTCGCTTTTTGTGCG TTTCTTTGTGCGTATGGATAATGTAACGGAGCGTATGCTTGGTGTG GTAAACGACCAAATGACAGAAAAAGAACGCGAAGCTGCACTAAACA GAGAAATGGCGAAAATTCAAAAAGAGAATGACGGAGGTGGAAAAT ACGTAGTTTCCGTGCGTCCTTTCTTCCAAGGCAATGAATATTATTAC TTTGTTTATCAAGATTTTAAAGATGTTCGATTGGTAGGTACCCCAAC CGAAAGCATCGGGAAATTCGGTGGAGATACTGACAATTGGGAATG GCCTCGCCAAACTGGTGATTTTTCTCTATTCCGTGTATATACCGACA AAAACGGAAATGCAGCAGAATATTCACCTGAAAACATTCCTTTAAG GACTGAAAATTATTTAAAAATCAACATCGGAGGAGTTAAAGAAAATG ATTTTGCAATGATTTTGGGCTATCCTGGGCGTACTAACCGTTGGGT TCCTTCGTTTTGGGTGGATCAAAATGTAAAATATGCTTATCCGGCTT GGGTTGAAGCCTCAAAAACAGCAATGGATGCTATGAAACCGTTTAT GGATGCCGACCCTGCCATCCGCTTAATGTATGCTTCTAAATATGCA CAAATTGCCAATTATTGGAAAAACAGACAAGGTATGATAGAGGCAC TCACAGCACATAAAACCGCCGAAACCAAACGCAAATACGAGAAAAA GTTTGCTAAGTGGGCCAATAGAAAGAAAAATCGTGACCAATATGCA CAGGTTTTACCCACTATGCAGAATTATTTTGAGAAAACGAATCAATA TTCGGCAGACCGAAATTATCTGATGTCTCTATTACGTGCTACTAATT TTGCTACTGTACCCAATACCATTGCTTTACAATTGCAACGCTTAGAG CAACTCCCCAAAGAGCAACAAACAGCCCTACTTGAACGCTTCAAAA ACCTCAGTGCTATTTATGACGAAATGCATATCCCTACGGAAAAAGC CATTTTAACAAATGGACTTACATTGTATGCATCAAAAGCAGCAAATG TATCGCAGGATGTAAAACAAATTTACACTGATTATCAAGGTGATTTC AAAAAATTTGTAGAAGAAGCCTTTGGAAAAGTATTTTTGCTTCCAA AGAAAAGTATATCCAATTTTTGGAAAATCCTGATGCAAAAATATTGG AAAACGACCCGCTTACACAACTTTCTAAGGCATTAATGACTGCATA CAACCAGAAATCAGAAAGCATTACTTCACTTGAAGATGGCTATCAA AAAGCATATCGTAAATTTGTTCAAGGAATGCGTAAATCTGGGGTAA GTCGTATTCAATACCCCGATGCCAACTCTACATTACGATTGACTTAC GGTACAGTTAAAGCCCTGCCCAATGACGAACGTAATGCAGATGCAA GCATTAACAACTATACTACTTTCAAGTCAATGGTTGCCAAATATAAA CCTAATGACCCCGAATTCGATATGCCTAAACGTATGGTCGAGATGT ACGAAAAAAATGATTTTGGAAGATATTTGGACAAAGACGGAACAAT GCACGTAAACTTCCTGACCAATAACGATATTACGGGCGGAAATTCA GGTTCGCCGGTAATGAATGGAAAAGGAGAACTTATCGGTTTGGCTT TTGATGGTAACATTGAAGCGATGGCTGGAGACGTTATTTTTGATGC AAAACTACAACGTACCATCAACGTAGATATTCGTTATGTGCTTTGGG TAATTGATAAATTTTCTGGAGCCAAACATATTGTTGATGAAATGACT TTGGTAGAATAG |
| SEQ ID NO:3 | amino acid | AIVQFNGGCTASIISRDGLVLTNHHCGYGQIAEVSSPENNYLRDGFWA ATHKDE |
| SEQ ID NO:4 | amino acid | DVRLVGTPTESIGKFGGDTDNWEWPRQTGDFSLFRVYTDKNGNAAE YSPENIPLRTENYLKINIGGVKENDFAMILGYPGRTNR |
| SEQ ID NO:5 | amino acid | PDANSTLRLTYGTVKALPNDERNADASINNYTTFKSMVAKYKPNDPEF DMPKRMVEMYEKNDFGRYLDKDGTMHVNFLTNNDITGGNSGSPVMN GKGELIGLAFDGNIEAMAGDVIFDAKLQRTINVDIRYVL |
| SEQ ID NO:6 | amino acid | TNHHCGYG |
| SEQ ID NO:7 | amino acid | NNDITGGNSGSP |

The term "amino acid" as used herein generally refers to a molecule that contains both amine and carboxyl functional groups. In biochemistry, this term particularly refers to alpha-amino acids with the general formula H₂NCHRCOOH, where R is an organic substituent. In the alpha-amino acids, the amino and carboxylate groups are attached to the same carbon, i.e., the α-carbon. The term includes the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, norvaline, norleucine and ornithine. The term includes both D- and L-amino acids. L-amino acids are preferred.

The present disclosure also relates to "functionally active variants or fragments" of the DPP7 peptide disclosed herein. The expression comprises functionally active variants of the DPP7 peptide, functionally active fragments of the DPP7 peptide, as well as functionally active variants of fragments of the DPP7 peptide.

The term "fragment" of a protein, polypeptide, or peptide generally refers to N-terminally and/or C-terminally deleted or truncated forms of said protein, polypeptide or peptide. The term encompasses fragments arising by any mechanism, such as, without limitation, by alternative translation, exo- and/or endo-proteolysis and/or degradation of said peptide, polypeptide or protein, such as, for example, *in vivo* or *in vitro,* such as, for example, by physical, chemical and/or enzymatic proteolysis. Without limitation, a fragment of a protein, polypeptide, or peptide may represent at least about 5% (by amino acid number), or at least about 10%, e.g., 20% or more, 30% or more, or 40% or more, such as preferably 50% or more, e.g., 60% or more, 70% or more, 80% or more, 90% or more, or 95% or more of the amino acid sequence of said protein, polypeptide, or peptide, e.g., a DPP7 peptide as set forth in SEQ ID NO: 1.

For example, a fragment of a protein, polypeptide, or peptide may include a sequence of 5 or more consecutive amino acids, 10 or more consecutive amino acids, 20 or more consecutive amino acids, 30 or more consecutive amino acids, e.g., 40 or more consecutive amino acids, such as for example 50 or more consecutive amino acids, 60 or more, 70 or more, 80 or more, 90 or more, 100 or more, 200 or more, 300 or more, 310 or more, 320 or more, 330 or more, 340 or more, 350 or more, 360 or more, 370 or more, 380 or more, or 390 or more consecutive amino acids of the corresponding full-length protein or polypeptide, e.g a DPP7 peptide as set forth in SEQ ID NO: 1.

In an embodiment, a fragment of a protein, polypeptide, or peptide may be N-terminally and/or C-terminally truncated by between 1 and about 20 amino acids, such as by between 1 and about 15 amino acids, or by between 1 and about 10 amino acids, or by between 1 and about 5 amino acids, compared with the corresponding full-length protein or polypeptide, e.g., a DPP7 peptide as set forth in SEQ ID NO: 1.

The term "variant" of a protein, polypeptide or peptide generally refers to proteins, polypeptides or peptides the amino acid sequence of which is substantially identical (i.e., largely but not wholly identical) to the sequence of the protein, polypeptide, or peptide, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical to the sequence of the protein, polypeptide, or peptide, e.g., a DPP7 peptide as set forth in SEQ ID NO: 1.

Preferably, a variant may display such degrees of identity to a recited protein, polypeptide or peptide when the whole sequence of the recited protein, polypeptide or peptide is queried in the sequence alignment (i.e., overall sequence identity). Sequence identity may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62 (Henikoff et al., 1992, Proc. Natl. Acad. Sci., 89:10915-10919), cost to open a gap = 11, cost to extend a gap = 1, expectation value = 10.0, word size = 3).

An example procedure to determine the percent identity between a particular amino acid sequence and the amino acid sequence of a query polypeptide (e.g., a DPP7 peptide as set forth in SEQ ID NO: 1) will entail aligning the two amino acid sequences using the Blast 2 sequences (BI2seq) algorithm, available as a web application or as a standalone executable programme (BLAST version 2.2.31+) at the NCBI web site (www.ncbi.nlm.nih.gov), using suitable algorithm parameters. An example of suitable algorithm parameters include: matrix = Blosum62, cost to open a gap = 11, cost to extend a gap = 1, expectation value = 10.0, word size = 3). If the two compared sequences share homology, then the output will present those regions of homology as aligned sequences. If the two compared sequences do not share homology, then the output will not present aligned sequences. Once aligned, the number of matches will be determined by counting the number of positions where an identical amino acid residue is presented in both sequences. The percent identity is determined by dividing the number of matches by the length of the query polypeptide, followed by multiplying the resulting value by 100. The percent identity value may, but need not, be rounded to the nearest tenth. For example, 78.11, 78.12, 78.13, and 78.14 may be rounded down to 78.1, while 78.15, 78.16, 78.17, 78.18, and 78.19 may be rounded up to 78.2. It is further noted that the detailed view for each segment of alignment as outputted by BI2seq already conveniently includes the percentage of identities.

A variant of a protein, polypeptide, or peptide may be a homologue (e.g., orthologue or paralogue) of said protein, polypeptide, or peptide. As used herein, the term "homology" generally denotes structural similarity between two macromolecules, particularly between two proteins or polypeptides, from same or different taxons, wherein said similarity is due to shared ancestry.

A variant of a protein, polypeptide, or peptide may comprise one or more amino acid additions, deletions, or substitutions relative to (i.e., compared with) the corresponding protein or polypeptide, e.g., a DPP7 peptide as set forth in SEQ ID NO: 1.

For example, a variant (substitution variant) of a protein, polypeptide, or peptide may comprise up to 50 (e.g., not more than one, two, three, four, five, six, seven, eight, nine, ten, 12, 15, 20, 25, 30, 35, 40, or 50) conservative amino acid substitutions relative to (i.e., compared with) the corresponding protein or polypeptide, e.g. a DPP7 peptide as set forth in SEQ ID NO: 1.

A conservative amino acid substitution is a substitution of one amino acid for another with similar characteristics. Conservative amino acid substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The nonpolar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (i.e., basic) amino acids include arginine, lysine and histidine. The negatively charged (i.e., acidic) amino acids include aspartic acid and glutamic acid. Any substitution of one member of the above-mentioned polar, basic, or acidic groups by another member of the same group can be deemed a conservative substitution. By contrast, a non-conservative substitution is a substitution of one amino acid for another with dissimilar characteristics.

Alternatively or in addition, for example, a variant (deletion variant) of a protein, polypeptide, or peptide may lack up to 20 amino acid segments (e.g., one, two, three, four, five, six, seven, eight, nine, ten, 11, 12, 13, 14, 15, 16, 17, 18, 19, or 20 segments) relative to (i.e., compared with) the corresponding protein or polypeptide, e.g., a DPP7 peptide as set forth in SEQ ID NO: 1. The deletion segment(s) may each independently consist of one amino acid, two contiguous amino acids or three contiguous amino acids. The deletion segments may be non-contiguous, or two or more or all of the deletion segments may be contiguous.

As disclosed herein, the DPP7 peptide may also be fused with one or more internal and/or terminal (i.e., N- and/or C-terminal) irrelevant or heterologous amino acid sequences (i.e., fusion protein). A heterologous sequence can be, for example a sequence used for purification of the recombinant protein (e.g., FLAG, polyhistidine (e.g., hexahistidine), hemagluttanin (HA), glutathione-S-transferase (GST), or maltose-binding protein (MBP)). Heterologous sequences can also be proteins useful as diagnostic or detectable markers, for example, luciferase, green fluorescent protein (GFP), or chloramphenicol acetyl transferase (CAT). In some embodiments, the fusion protein may contain a signal sequence from another protein. In certain host cells (e.g., yeast host cells), expression and/or secretion of the target protein can be increased through use of a heterologous signal sequence. In certain embodiments, the fusion protein can contain a carrier (e.g., KLH) useful, e.g., in eliciting an immune response (e.g., for antibody generation; see below) or endoplasmic reticulum or Golgi apparatus retention signals. Heterologous sequences can be of varying length and in some cases can be a longer sequences than the full-length protein or polypeptide to which the heterologous sequences are attached.

Where the present specification refers to or encompasses variants and/or fragments of proteins, polypeptides or peptides, this denotes variants or fragments which are functionally active or functional, i.e., which at least partly retain the biological activity or intended functionality of the respective or corresponding proteins, polypeptides, or peptides. By means of an example and not limitation, a functionally active variant or fragment of DPP7 peptide as disclosed herein shall at least partly retain the biological activity of the DPP7 peptide. For example, it may retain one or more aspects of the biological activity of the DPP7, such as its serine protease activity. Preferably, a functionally active variant or fragment may retain at least about 20%, e.g., at least about 25%, or at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared with the corresponding protein, polypeptide, or peptide. Reference to the "activity" of a protein, polypeptide, or peptide such as the DPP7 peptide may generally encompass any one or more aspects of the biological activity of the protein, polypeptide, or peptide, such as without limitation any one or more aspects of its biochemical activity, enzymatic activity, signalling activity, interaction activity, ligand activity, and/or structural activity, e.g., within a cell, tissue, organ or an organism. By means of an example and not limitation, reference to the activity of the DPP7 peptide or functionally active variant or fragment thereof may particularly denote its activity as a serine protease, i.e., its ability to cleave and inactivate a coagulation factor, preferably FX. Where the activity of a given protein, polypeptide, or peptide such as the DPP7 peptide can be readily measured in an established assay, e.g., an enzymatic assay (such as, for example, by a fluorimetric assay), a functionally active variant or fragment of the protein, polypeptide, or peptide may display activity in such assays, which is at least about 20%, e.g., at least about 25%, or at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the activity of the respective or corresponding protein, polypeptide, or peptide.

For example, the protease activity of the DPP7 peptide or functionally active variant or fragment thereof on a coagulation factor can be measured by a variety of methods for measuring clot formation, including but not limited to a chromogenic FX assay, activated partial thromboplastin time assay, prothrombin time, activated clotting time, thromboelastography, thrombin generation assay, dilute Russel's viper venom time, ecarin clotting time, kaolin clotting time, International Normalized Ratio (INR), fibrinogen testing (Clauss), thrombin time (TCT), mixing time. These and other methods for measuring clot formation are known to the skilled person.

By "protease" is meant enzyme that performs proteolysis, that is, begins protein catabolism by hydrolysis of the peptide bonds that link amino acids together in a polypeptide chain. Different classes of proteases can perform the same reaction by completely different catalytic mechanisms. Proteases can be classified into several groups based on the catalytic mechanism they use, for example, serine proteases use a serine alcohol, cysteine proteases use a cysteine thiol, threonine proteases use a threonine secondary alcohol, aspartic proteases use an aspartate carboxylic acid, glutamic proteases use a glutamate carboxylic acid, glutamic proteases use a glutamate carboxylic acid, metalloproteases use a metal and asparagine peptide lyases use an asparagine to perform an elimination reaction in the absence of water. Alternatively, proteases may be classified by the optimal pH in which they are active, for example acid proteases, neutral proteases and basic (alkaline) proteases, or according to the sites at which they catalyse the cleavage of proteins, for example exopeptidases which target the terminal ends of proteins and endopeptidases which target sites within proteins. The terms "proteolytic enzyme", "protease", "proteinase" and "peptidase" can be used interchangeably. Protease activity may be determined by a variety of protease activity assays known to the skilled person. For example, colorimetric protease assays using a fluorescently labelled protease substrate such as fluorescein isothiocyanate-labelled casein.

In certain examples, a functionally active variant or fragment of the DPP7 peptide may have at least 25% (e.g., at least 30%, at least 40%, at least 50%, at least 60%, at least 70%, at least 75%, at least 80%, at least 85%, at least 90%, at least 95%, at least 98%, at least 99%, at least 100%, or even greater than 100%) of the DPP7 enzymatic activity of the DPP7 peptide as set forth in SEQ ID NO: 1. The functional variant or fragment can generally, but not always, be comprised of a continuous region of the protein, wherein the region has functional activity. The amino acid residues forming the active site of the DPP7 peptide are located in three different conserved regions (Rouf et al., Discrimination based on Gly and Arg/Ser at position 673 between dipeptidyl-peptidase (DPP) 7 and DPP11, widely distributed DPPs in pathogenic and environmental gram-negative bacteria, Biochemie, 2013, 95:824-832). The first region ranges from an alanine to a glutamic acid at an amino acid position corresponding to the position of amino acid 62 and 115 of the DPP7 polypeptide as set forth in SEQ ID NO: 1, respectively, and corresponding to the amino acid sequence as set forth in SEQ ID NO:3. The second region ranges from an aspartic acid to an arginine at an amino acid position corresponding to the position of amino acid 193 and 276 of the DPP7 polypeptide as set forth in SEQ ID NO: 1, respectively, and corresponding to the amino acid sequence as set forth in SEQ ID NO:4. The third region ranges from a proline to a leucine at an amino acid position corresponding to the position of amino acid 562 and 694 of the DPP7 polypeptide as set forth in SEQ ID NO: 1, respectively, and corresponding to the amino acid sequence as set forth in SEQ ID NO:5. These regions comprise subregions with highly similar amino acid residues, these subregions comprise amino acid sequences corresponding to the amino acid sequence as set forth in SEQ ID NO:6 and SEQ ID NO:7. The essential amino acids forming the catalytic triad of the serine protease family are a histidine, an aspartic acid and a serine at an amino acid position corresponding to the position of amino acid 86, 193 and 649 of the DPP7 polypeptide as set forth in SEQ ID NO: 1, respectively. Furthermore, a glycine instead of an arginine at an amino acid position corresponding to the position of amino acid 667 of the DPP7 polypeptide as set forth in SEQ ID NO: 1 is critical to define substrate specificity of DPP7 (glycine) and DPP11 (arginine) (Figure 1).

More particularly, the first region comprises the amino acid sequence as set forth in SEQ ID NO:3, of which a highly conserved subregion comprises the amino acid sequence as set forth in SEQ ID NO:6.

More particularly, the second conserved region comprises the amino acid sequence as set forth in SEQ ID NO:4.

More particularly, the third conserved region comprises the amino acid sequence as set forth in SEQ ID NO:3, of which a highly conserved subregion comprises the amino acid sequence as set forth in SEQ ID NO:7.

Candidate functional variants or fragments of DPP7 peptides can therefore be produced by one skilled in the art using well established methods, such as homology modelling and computational engineering, and tested for the desired enzymatic activity.

In particular embodiments, the DPP7 peptide for use according to the invention comprises an amino acid sequence which has at least 90%, preferably at least 95%, most preferably 100% identity with the amino acid sequence set forth in SEQ ID NO:1, or functionally active variants or fragments thereof.

In particular embodiments, the DPP7 peptide for use according to present invention comprises:
(a)an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(b)an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(c)an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1 ;
or functionally active variants or fragments thereof.

Further, unless otherwise apparent from the context, reference herein to any nucleic acid, peptide, polypeptide or protein and variants or fragments thereof may generally also encompass altered forms of said nucleic acid, peptide, polypeptide or protein and variants or fragments such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

Incubation of DPP7 with FX surprisingly lead to an increase in clotting time as DPP7 degrades FX heavy and light chain from the N-terminus and renders FX dysfunctional.

The terms 'coagulation factor X', 'Factor X' and 'FX' as used herein refer to a plasma serine protease involved in the blood coagulation cascade. FX is a two-chain protein consisting of a light chain (17kDa) and a heavy chain (45kDa) held together by one or more di-sulphide bonds. The light chain contains an N-terminal gamma-carboxyglutamic acid domain (Gla domain and two epidermal growth factor-like domains (EGF1 and EGF2), while the heavy chain consists of an N-terminal activation peptide (AP, N-terminal 52 amino acid residues) and a serine-protease domain. FX is activated by hydrolysis into an active enzyme FXa. More particularly, the FX heavy chain is cleaved during coagulation by both FIX (with cofactor FVIII) and FVII (with its cofactor TF). The activation peptide is released when FX is activated to FXa, but the heavy and light chains remain covalently linked following activation. FXa acts by cleaving prothrombin in two places (firstly, an Arg-Thr and secondly, an Arg-IIe bond), which yields the active thrombin. FX shares extensive sequence homology with FVII and FIX.

The terms 'coagulation factor VII', 'Factor VII' and 'FVII' as used herein refer to a plasma serine protease involved in the blood coagulation cascade. FVII is a single chain, vitamin-K dependent, plasma glycoprotein. FVII is proteolytically activated to the serine protease FVIIa during coagulation. FVII can be activated by thrombin, FIXa, FXa or FXIIa. The activation results in cleavage of the single chain molecule on the COOH-terminal side of arginine-152 to produce an NH2-terminal derived light chain, comprising a Gla domain as well as two EGF domains, and a COOH-terminal derived heavy chain which remain covalently associated by a single disulfide bond. The heavy chain region of FVIIa contains the catalytic domain. FVIIa can become part of the Xase enzyme complex, which catalyses the conversion of FIX to FIXa and FX to FXa. FVII shares extensive sequence homology with FX and FIX.

The terms 'coagulation factor IX', 'Factor IX' and 'FIX' as used herein refer to a plasma serine protease involved in the blood coagulation cascade. FIX is a single chain, vitamin-K dependent, plasma glycoprotein. FIX is proteolytically activated to the serine protease FIXa during coagulation. FIX can be activated by thrombin, FXIa or the FVIIa/TF/phospholipid complex to the active two-chain form FIXa. More particularly, the activation results in cleavage of the single chain molecule on the COOH-terminal side to produce an NH2-terminal derived light chain, comprising a Gla domain as well as two EGF domains, and a COOH-terminal derived heavy chain which remain covalently associated by a single disulfide bond. The heavy chain region of FIXa contains the catalytic domain. FIXa is the catalytic component of the intrinsic factor Xase complex, which catalyses the conversion of FX to FXa. FIX shares extensive sequence homology with FVII and FIX. FIX is inhibited by antithrombin.

Reduced activity, delayed activation or complete inactivation of FX, FVII and FIX can be achieved by removing amino acid residues from the single, heavy and/or light chain or by preventing the binding of FIX and/or FVII and their cofactors to FX.

The activity of FX, FVII and FIX can be determined by a variety of methods for measuring clot formation, including but not limited to a chromogenic FX(a) assay, activated partial thromboplastin time assay, prothrombin time, activated clotting time, thromboelastography, thrombin generation assay, dilute Russel's viper venom time, ecarin clotting time, kaolin clotting time, International Normalized Ratio (INR), fibrinogen testing (Clauss), mixing time. These and other methods for measuring clot formation are known to the skilled person.

In particular embodiments, the DPP7 peptide, or functionally active variants or fragments according to present invention reduces or eliminates the activity of coagulation FX, FVII and/or FIX. Preferably, the degree of inactivation is at least about 20%, e.g., at least about 25%, or at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% of the original biological activity.

DPP7 acts by cleaving factor X in an area called the GLA domain. This domain is essential for the activity of factor X within the prothrombinase complex. By cleaving, and thus inactivating the Gla domain, DPP7 makes factor X inefficient.

In particular embodiments, the DPP7 peptide, or functionally active variants or fragments according to the invention cleaves at least 1, at least 2, at least 3, at least 4, at least 5, at least 6, at least 7, at least 8, at least 9, at least 10, at least 20, at least 30, at least 40, at least 50, at least 60, at least 70, at least 80, at least 90 or at least 100 consecutive amino acid residues of the N-terminus and/or C-terminus of the heavy and/or light chain of FX, preferably at least one, more preferably at least 10 amino acid residues of the N-terminus of the heavy chain and one or more amino acid residues of the N-terminus of the light chain. Removal of only few residues from the FX HC and LC N-terminus is sufficient to inactivate FX. For example, the absence of all, but three amino acids of the AP has been shown to hamper the activation of FX by intrinsic Xase (Duffy et al., Intrinsic pathway activation of factor X and its activation peptide-deficient derivative, factor Xdes-143-191. J Biol Chem. 1992 ; 267 :7821-7). Successful cleavage of FX by the DPP7 peptide can be determined by, for example, silver staining, western blot analysis using antibodies specifically binding the N-terminus or C-terminus of the heavy or light chain of FX or by N-terminal and/or C-terminal sequencing of the cleaved FX.

In particular embodiments, the DPP7 peptide, or functionally active variants or fragments according to the invention cleaves at least 1, at least 2, at least 3, at least 4, at least 5, at least 6, at least 7, at least 8, at least 9, at least 10, at least 20, at least 30, at least 40, at least 50, at least 60, at least 70, at least 80, at least 90 or at least 100 consecutive amino acid residues of the FVII and/or FIX single chain, preferably at least 1 amino acid residues. Successful cleavage of FVII and/or FIX by the DPP7 peptide can be determined by, for example, silver staining, western blot analysis using antibodies specifically binding the N-terminus or C-terminus of the single chain of FVII and/or FIX or by N-terminal or C-terminal sequencing of the cleaved FVII and/or FIX.

In particular embodiments, the DPP7 peptide, or functionally active variants or fragments according to the invention cleaves at least 1, at least 2, at least 3, at least 4, at least 5, at least 6, at least 7, at least 8, at least 9, at least 10, at least 20, at least 30, at least 40, at least 50, at least 60, at least 70, at least 80, at least 90 or at least 100 consecutive amino acid residues of FX alone; both FX and FVII; both FX and FIX; or FX, FVII and FIX.

Inhibition of the synthesis or activity of FX, FVII and/or FIX prevents blood clots developing or worsening and therefore, FX, FVII and/or FIX inhibitors can act as anticoagulants. Anticoagulants are typically used to treat or prevent a number of medical conditions caused by overactive clotting. A non-limiting list of medical conditions is atrial fibrillation, pulmonary embolism, deep vein thrombosis, venous thromboembolism, congestive heart failure, stroke, coronary syndrome, peripheral arterial occlusion, myocardial infarction, and/or genetic or acquired hypercoagulability. Anticoagulants may also be used to reduce the chance of blood clots forming during open-heart surgery or bypass surgery or after the replacement of a heart valve. Furthermore, low doses of anticoagulants may be given to prevent blood clots in patients who must stay in bed for a long time after certain types of surgery. Inhibiting FX, FVII and/or FIX before they are activated to FXa, FVIIa and/or FIXa, is especially important since residual FX, FVII and/or FIX can still be activated, providing a sufficient haemostasis once it is required (e.g. at a site of injury). The reduction of the thrombotic potential, which can be linked to the level of FXa, FVIIa and/or FIXa, provides a sufficient anticoagulant effect. Current anticoagulants only target activated coagulation factors, while the DPP7 peptide, or functionally active variants or fragments according to the invention can inactivate FX, FVII and/or FIX.

Haemostasis disorders and the activity of the anticoagulant may be determined by a variety of methods for measuring clot formation, including but not limited to a FX(a) chromogenic activity assay, FVIII(a) chromogenic activity assay, FIX(a) chromogenic activity assay, activated partial thromboplastin time assay, prothrombin time, thrombin time, activated clotting time, thromboelastography, thrombin generation assay, reptilase time, dilute Russel's viper venom time, ecarin clotting time, kaolin clotting time, International Normalized Ratio (INR), fibrinogen testing (Clauss), thrombin time (TCT), mixing time, and euglobulin lysis time. These methods help to determine various coagulation parameters, and are known to the skilled person.

In particular embodiments, the DPP7 peptide, or functionally active variants or fragments thereof, for use according to the invention, as an anticoagulant.

In particular embodiments, the DPP7 peptide, or functionally active variants or fragments thereof, for use according to the invention, in the treatment and/or prevention of a medical disorder chosen from the list comprising atrial fibrillation, pulmonary embolism, deep vein thrombosis, venous thromboembolism, congestive heart failure, stroke, coronary syndrome, peripheral arterial occlusion, myocardial infarction and/or genetic or acquired hypercoagulability.

The terms "treat" or "treatment" encompass both the therapeutic treatment of an already developed disease or condition. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "Treatment" can also mean prolonging survival as compared to expected survival if not receiving treatment.

The terms "prevent" or "prevention" encompass both the delaying or averting a disease or condition that has not developed yet. However, there are indications that the disease or condition would develop without administering the drug.

Alternatively, anticoagulants can be used for devices which are intended to come into contact with blood *in vitro* or *in vivo.* In the field of *in vitro* diagnostics, containers for collecting, preserving and/or storing blood often comprise an anticoagulant to retain the blood in a fluid state for, for example, haematological testing or to obtain suitable plasma for coagulation and clinical chemistry analysis. Examples of such containers are blood collection tubes and blood transfusion bags. Anticoagulants can also be used to coat, for example, but not limited to, indwelling devices (e.g. stents, cathether, balloons, vascular prostheses, artificial blood vessels, artificial skins, transdermal devices, adhesion-preventing materials, and wound dressings), blood drawing devices (e.g. needles, syringes) and renal dialysis equipment.

Another aspect of the invention is the *in vitro* use of a DPP7 peptide, or functionally active variants or fragments thereof, in the cleavage and inactivation of coagulation factor X, VII and/or IX, preferably factor X.

Well-regulated haemostasis is crucial for health and both inadequate coagulation, such as in the inherited disorder haemophilia, and excessive coagulation, as occurs in thrombophilia, can have drastic consequences such as hemorrhage and thrombosis. Haemostasis disorders can be separated into two main groups: inherited or acquired and are further classified in coagulation factor deficiencies, platelet disorders, vascular disorders and fibrinolytic defects. Non-limiting examples are Fibrinogen deficiency, Prothrombin deficiency, Factor V deficiency, Combined Factor V and VIII deficiency, Factor VII deficiency, Factor VIII deficiency (Haemophilia A), Factor IX deficiency (Haemophilia B), Factor X deficiency, Factor XI deficiency, Factor XIII deficiency, Glanzmann's thrombasthenia, Bernard Soulier Syndrome, Wiskott-Aldrich Syndrome and Leukocyte Adhesion deficiency.

Haemostasis disorders that lead to overactive clotting are mostly treated with a coagulation inhibitor. The coagulation inhibitor (also referred to herein as anticoagulant) is a molecule that inhibits coagulation process. With the involvement of the direct oral anticoagulants, several antidotes have emerged in the last years. The DPP7 peptide according to present invention can serve in the production of an antidote against coagulation inhibitors, more particularly, direct oral anticoagulants (DOACs), even more particularly FX(a), FVII(a) or FIX(a) inhibitors, preferably FXa inhibitors. Non-limiting examples of FXa inhibitors are rivaroxaban, apixaban, edoxaban, betrixaban, otamixaban, fondaparinux, idraparinux and idrabiotaparinux. By cleaving the Gla domain, the DPP7 peptide according to present invention generates an inefficient/truncated FXa, FVIIa and/or FIXa which, once administered to the patient, will be able to compete with endogenous FXa, FVIIa and/or FIXa for the different FXa, FVIIa and/or FIXa inhibitors, respectively. By fixing the FXa, FVIIa or FIXa inhibitor, the truncated FXa, FVIIa and/or FIXa will be able the restore normal haemostasis.

In particular embodiments, the *in vitro* use of the DPP7 peptide, or functionally active variants or fragments thereof, according to the invention for producing of an anticoagulant reversal agent, wherein said anticoagulant is a factor X(a), factor VII(a) or factor IX(a) inhibitor, preferably a factor Xa inhibitor.

Coagulation factor-depleted plasmas are typically normal human plasmas from which coagulation factors have been removed by selective immunoaffinity chromatography. In particular, immuno-depletion is a method for removing a target molecule from a mixture. Depletion begins by adding an antibody targeting the molecule of interest and then following with a solid phase material to bind the antibody. Examples include beads (agarose or magnetic) conjugated to protein A, protein G, avidin or anti-IgG. After mixing, the samples are centrifuged and the supernatant is saved as the depleted product. Thus, several steps are required which increase the turn-around time, complicate the production process and increase the cost. In addition, depending on the antibody, depletion may not be optimal (some reported depletion rate between 90 and 95%) and not always specific which means that this process can introduce slight deficiencies of other factors. Hence, the use of these depleted plasma is generally limited to specific techniques which are insensitive to the depletion of other factors.

Coagulation factor depleted plasmas are key components for the specific diagnosis of haemophilia or haemostasis abnormalities and are typically used as substrate plasmas in the quantitative determination of a coagulation factor activity in plasmas using a clot-based assay. For example, Factor X activity in patient plasma can determined *in vitro* through the use of a modified prothrombin time test (PT). Patient plasma is diluted, mixed with plasma deficient in Factor X and a PT test performed. The result is interpolated using a reference curve generated with dilutions of calibrator plasma mixed with the Factor X deficient plasma. Correction of the clotting time of the deficient plasma is proportional to the concentration (% activity) of Factor X in the patient plasma, intrapolated from the calibartion curve.

Since the Applicants demonstrated that the DPP7 peptide can inactivate FX, FVII and FIX, preferably FX, the DPP7 peptide can also be used for depleting fluids, preferably body fluids such as plasma obtained from a subject, of coagulation FX, FVII and/or FIX, preferably FX. In particular, the DPP7 peptide may be used for engineering FX-, FVII- and/or FIX-deficient plasma for human diagnostic purposes. To achieve a depletion of FX, FIV and/or FVII, DPP7 can be immobilized, for example, by attachment to the surface of an inert, insoluble material such as alginate, chitosan, chitan, collagen, carrageenan, gelatin, cellulose, starch, pectin, sepharose, synthetic polymers or any other inert, insoluble material known by the skilled in the art, preferably bead-shaped. The fluid containing coagulation FX, FIV and/or FVII can be put temporarily into contact with the DPP7-coated material in a container, preferably a test tube or column. For example, chromatography, centrifugation, a magnet or any other method known by the skilled person can be used to separate the fluid from the DPP7-coated material after contact. Furthermore, a chromogenic substrate for FXa, FIXa and/or FVIIa, TF, silica, celite, kaolin or ellagic acid, calcium chloride and/or phospholipids to verify the efficiency of depletion may be used in the depletion. It will be clear to the skilled in the art that other methods may be used to obtain FX-, FVII- and/or FIX-depleted plasma using the DPP7 peptide according to present invention.

FX-, FVII- and/or FIX-deficient plasma can be used, for example, to detect FX, FIX or FVII deficiencies in patients. The plasmas depleted for FX, FIX or FVII using the DPP7 peptide according to present invention are of high-quality. Furthermore, the use of the DPP7 peptide according to present invention is easier and more efficient than existing methods. Furthermore, given that the DPP7 peptide according to present invention could be easily produced, the depleted plasma obtained by use of said DPP7 peptide is cheaper than existing commercial depleted plasmas.

In particular embodiments, the *in vitro* use of the DPP7 peptide, or functionally active variants or fragments thereof, according to the invention, for depleting a plasma sample of coagulation factor X, VII and/or FIX, preferably factor X.

As used herein, the term "plasma sample" refers to a plasma sample obtained from a subject, preferably a healthy subject.

In particular embodiments, the *in vitro* use of the DPP7 peptide, or functionally active variants or fragments thereof, according to the invention, as an anticoagulant.

In particular embodiments, the *in vitro* use of the DPP7 peptide, or functionally active variants or fragments thereof, according to the invention, in medical devices.

The term "medical devices" as used herein refers to any instrument, apparatus, appliance, software, material or other article, whether used alone or in combination, to be used specifically for diagnostic and/or therapeutic purposes.

In particular embodiments, the *in vitro* use of the DPP7 peptide, or functionally active variants or fragments thereof, according to the invention, wherein said DPP7 peptide is bacterial DPP7.

In particular embodiments, the *in vitro* use of the DPP7 peptide, or functionally active variants or fragments thereof, according to the invention, wherein said DPP7 peptide is *Porphyromonas gingivalis* DPP7, *Capnocytophaga canimorsus* DPP7, *Capnocytophaga canis nov. sp.* DPP7 or *Capnocytophaga cynodegmi* DPP7, preferably *Capnocytophaga canimorsus* DPP7.

In particular embodiments, the *in vitro* use of the DPP7 peptide according to the invention; wherein said peptide comprises an amino acid sequence which has at least 90%, preferably at least 95%, most preferably 100% identity with the amino acid sequence set forth in SEQ ID NO:1, or functionally active variants or fragments thereof.

In particular embodiments, the *in vitro* use of the DPP7 peptide according to the invention; wherein said peptide comprises:
(a)an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(b)an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(c)an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1 ;
or functionally active variants or fragments thereof.

Another aspect of the invention relates to a kit comprising a DPP7 peptide, or functionally active variants or fragments thereof, wherein said peptide or functionally active variants or fragments thereof, has anticoagulant activity, preferably by inhibiting clotting FX, FVII and/or FIX, preferably wherein said DPP7 peptide comprises an amino acid sequence which has at least 90%, preferably at least 95%, most preferably 100% identity with the amino acid sequence set forth in SEQ ID NO:1, or functionally active variants or fragments thereof.

The kit comprising a DPP7 peptide may be a diagnostic kit further comprising ready-to use substrate solutions, wash solutions, dilution buffers and additional compounds (e.g. reptilase, phospholipid, diluted Russell's viper venom, calcium, calcium chloride, TF, silica, celite, kaolin or ellagic acid). The diagnostic kit may also comprise positive and/or negative control samples.

The kit may comprise carriers which allow visualization and/or a qualitative read-out of the clotting ability of the blood sample of the patient by, for example, spectrophotometry. The term 'carrier' as used herein refers to small containers in which the clotting reaction is performed. Typically, the minimum volume that the container can hold is larger than the minimum total volume required for the clotting reaction to occur. Optionally, these carriers may also allow for cascade testing. Non-limiting examples of carriers are translucent microtiter plates, translucent stripwells or translucent tubes.

The anticoagulant may be present in a solubilized form or a lyophilized form, if stable in that form. Lyophilized anticoagulant could be used to coat (microtiter) plates, stripwell plates or tubes, thereby simplifying the use of the diagnostic kit.

Preferably, the instructions included in the kit are unambiguous, concise and comprehensible to those skilled in the art. The instructions typically provide information on kit contents, how to collect the blood sample, methodology, experimental read-outs and interpretation thereof and cautions and warnings.

In particular embodiments, the kit comprises a DPP7 peptide, or functionally active variants or fragments thereof, wherein said peptide or functionally active variants or fragments thereof, has anticoagulant activity, preferably by inhibiting clotting factor X, VII and/or IX, wherein said DPP7 peptide comprises,
(a)an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(b)an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(c)an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1;
or functionally active variants or fragments thereof.

As described above, the DPP7 peptide according to present invention can be used for depleting plasma of FX, FIV and/or FVII, preferably FX. Coagulation factor-depleted plasmas are key components for the specific diagnosis of haemophilia or haemostasis abnormalities. For example, the correct diagnosis of a factor X deficiency is of major importance for patient-tailored treatment. Other source of possible clotting time prolongation need to be excluded. It is important to classify the factor X deficiency correctly with its severity according to the residual factor X activity (severe deficiency: <0.011U/mL; moderate deficiency: 0.01-0.051U/mL or mild deficiency: 0.05-0.401U/mL). Thus, the first important contributor to the precision of factor assays is the quality of the deficient plasma used in the test. Currently, commercially available, immuno-depleted factor deficient plasma are used. An important issue is the possible residual factor activity in the depleted plasma, as those with a residual factor activity of more than 0.01IU/mL may significantly affect the calibration curve, especially in the range below 0.03IU/mL where the accurate diagnosis is crucial for the patient. Currently, all batches of deficient plasma are checked and not unfrequently rejected for residual clotting factor activity.

By inactivating FX, FVII and/or FIX, the DPP7 peptide according to present invention produces, rapidly and easily a plasma completely depleted of FX, FVII and/or FIX respectively, which ensures a more accurate diagnosis.

Accordingly, present invention also relates to a kit comprising a plasma sample deficient of factor X, a plasma sample deficient of factor IV and/or a plasma sample deficient of factor VII, wherein said plasma samples are depleted from factor X, factor IV and/or factor VII using a DPP7 peptide, or functionally active variants or fragments thereof according to present invention.

Said kit comprising a plasma sample deficient of FX, a plasma sample deficient of FIV and/or a plasma sample deficient of FVII can be used to identify the missing clotting factor in patients that have a coagulation disorder.

In particular embodiments, the kit according to the invention comprises an array of plasma samples, each of them depleted in one specific factor. This allows identifying the missing clotting factor in patients more quickly.

The kit may be a diagnostic kit further comprising ready-to use substrate solutions, wash solutions, dilution buffers and additional compounds (e.g. reptilase, phospholipid, diluted Russell's viper venom, calcium, calcium chloride, TF, silica, celite, kaolin or ellagic acid).

The kit may comprise carriers which allow visualization and/or a qualitative read-out of the clotting ability of the blood sample of the patient by, for example, spectrophotometry.

Another aspect of the invention is a nucleic acid construct comprising a nucleic acid sequence which
(a) has at least 90%, preferably at least 95%, most preferably 100% identity with the nucleic acid sequence set forth in SEQ ID NO:2,
   and/or encodes a peptide comprising:
(b) an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(c) an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(d) an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1,
, operably linked to one or more control sequences capable of directing the expression of the polypeptide in a suitable expression host.

By "nucleic acid" is meant oligomers and polymers of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted sugars and/or one or more modified or substituted phosphate groups. Modifications of phosphate groups or sugars may be introduced to improve stability, resistance to enzymatic degradation, or some other useful property. A "nucleic acid" can be for example doublestranded, partly double stranded, or single-stranded. Where single-stranded, the nucleic acid can be the sense strand or the antisense strand. In addition, nucleic acid can be circular or linear. The term "nucleic acid" as used herein preferably encompasses DNA and RNA, specifically including RNA, genomic RNA, cDNA, DNA, provirus, pre-mRNA and mRNA. The term "nucleic acid construct" refers to an artificially constructed segment of nucleic acid which is going to be transferred into an expression host.

The nucleic acid construct comprises a nucleic acid sequence which is substantially identical (i.e., largely but not wholly identical) to the sequence of the nucleic acid sequence as set forth in SEQ ID NO: 2, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical to the nucleic acid sequence as set forth in SEQ ID NO: 2. Sequence identity may be determined using suitable algorithms for performing sequence alignments and determination of sequence identity as know *per se.* In present invention, the nucleic acid of SEQ ID NO:2 encodes the DPP7 peptide sequence as set forth in SEQ ID NO: 1.

In particular embodiments, the nucleic acid sequence which has at least 90%, preferably at least 95%, most preferably 100% identity with the nucleic acid sequence set forth in SEQ ID NO:2, operably linked to one or more control sequences capable of directing the expression of the polypeptide in a suitable expression host according to present invention encodes a DPP7 peptide, or a functionally active variant or fragment thereof, preferably a DPP7 peptide, or a functionally active variant or fragment thereof, as set forth in SEQ ID NO: 1.

By "encoding" is particularly meant that a nucleic acid sequence or part(s) thereof corresponds to another nucleic acid sequence in a template - transcription product (e.g., RNA or RNA analogue) relationship, or corresponds, by virtue of the genetic code of an organism in question, to a particular amino acid sequence, e.g., the amino acid sequence of one or more desired proteins or polypeptides.

In certain embodiments, the nucleic acid molecule may be codon optimized for expression of the DPP7 peptide or functionally active variant or fragment thereof in a host cell. For example in a bacterial cell, a fungal cell, including yeast cells, an animal cell, or a mammalian cell, including human cells and non-human mammalian cells. Preferably, the nucleic acid molecule is codon optimized for expression of the DPP7 peptide or functionally active variant or fragment thereof in a bacterial cell, more preferably in *Escherichia Coli, Porphyromonas gingivalis, Capnocytophaga canimorsus, Capnocytophaga canis, Capnocytophaga cynodegmi or Flavobacterium johnsoniae,* more preferably *Capnocytophaga canimorsus.*

An "operable linkage" is a linkage in which regulatory sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence. Hence, "operably linked" may mean incorporated into a genetic construct so that expression control sequences, such as a promoter, effectively control expression of a coding sequence of interest, such as the nucleic acid molecule as defined herein.

The nucleic acid sequence can also encompass a nucleic acid fragment encoding a tag. Tags can be used for various purposes, such as purification of the expressed peptide (e.g poly (His) tag), to assist proper protein folding (e.g. thioredoxin), separation techniques (e.g. FLAG-tag), or enzymatic or chemical modifications (e.g. biotin ligase tags, FIAsh), or detection (e.g. AviTag, Calmodulin-tag, polyglutamate tag, E-tag, FLAG-tag, HA-tag, His-tag, Myc-tag, S-tag, SBP-tag, Softag 1, Softag 3, Strep tag, TC tag, V5 tag, VSV-tag, Xpress tag, Isopeptag, SpyTag, Biotin Carboxyl Carrier Protein, Glutathione-S-transferase-tag, Green fluorescent protein tag, Halo-tag, Maltose binding protein-tag, Nus-tag, Thioredoxin-tag or Fc-tag). In the context of the present invention, their main purpose is purification.

Another aspect of the invention is a recombinant expression vector comprising the nucleic acid construct according to the invention, a promoter, and transcriptional, translational stop signals, and preferably, a selectable marker.

As used herein, the term "promoter" refers to a DNA sequence that enables a gene to be transcribed. A promoter is recognized by RNA polymerase, which then initiates transcription. Thus, a promoter contains a DNA sequence that is either bound directly by, or is involved in the recruitment, of RNA polymerase. A promoter sequence can also include "enhancer regions", which are one or more regions of DNA that can be bound with proteins (namely the trans-acting factors) to enhance transcription levels of genes in a gene-cluster. The enhancer, while typically at the 5' end of a coding region, can also be separate from a promoter sequence, e.g., can be within an intronic region of a gene or 3' to the coding region of the gene.

The promotor may be a constitutive or inducible (conditional) promoter. A constitutive promoter is understood to be a promoter whose expression is constant under the standard culturing conditions. Inducible promoters are promoters that are responsive to one or more induction cues. For example, an inducible promoter can be chemically regulated (e.g., a promoter whose transcriptional activity is regulated by the presence or absence of a chemical inducing agent such as an alcohol, tetracycline, a steroid, a metal, or other small molecule) or physically regulated (e.g., a promoter whose transcriptional activity is regulated by the presence or absence of a physical inducer such as light or high or low temperatures). An inducible promoter can also be indirectly regulated by one or more transcription factors that are themselves directly regulated by chemical or physical cues.

As used herein, the term "stop signal" refers to a transcription terminator or a translational stop codon. A transcription terminator is a fragment of nucleic acid sequence that indicates the end of a gene or operon in genomic DNA during transcription. This sequence provides signals in the newly synthesized mRNA that trigger processes which release the mRNA from the transcriptional complex, thereby mediating transcriptional termination. A stop codon is a nucleotide triplet within mRNA that does not code for an amino acid and thereby signals the termination of the synthesis of a protein. In RNA, this stop codon can be either UAG, UAA or UGA, wherein U is uracil, A is adenine and G is guanine.

As used herein, the term "selectable marker" refers to a marker gene, such that it can be determined whether or not the cell is capable of expressing the different nucleic acids of the nucleic acid construct based on the expression of this marker gene. Typically marker genes are used that confer resistance to a compound, which is added to the culture medium of the host cell, and will eliminate untransfected cells but not the transfected cells (positive selection, e.g. resistance to antibiotics). For example, selection antibiotics can be geneticin, zeocin, hygromycin B, puromycin or blasticidin. Antibiotics and their coding sequences are typically incorporated into the nucleic acid vector used for delivering genetic material into a target cell.

Another aspect of the invention is a recombinant host cell comprising the nucleic acid construct of claim 10, preferably contained on a vector or integrated into the host cell genome.

The term "vector" as used herein, is a tool that allows or facilitates the transfer of an entity from one environment to another. It is a replicon, such as a plasmid, phage, or cosmid, into which another DNA segment may be inserted so as to bring about the replication of the inserted segment. In present application, a vector is a nucleic acid molecule capable of transporting another nucleic acid to which it has been linked. One type of vector is a "plasmid" which refers to a circular double stranded DNA loop into which additional DNA segments can be ligated. Another type of vector is a phage vector. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" can be used interchangeably as the plasmid is the most commonly used form of vector.

Factors of importance in selecting a particular vector include *inter alia*: choice of recipient host cell, ease with which recipient cells that contain the vector may be recognised and selected from those recipient cells which do not contain the vector; the number of copies of the vector which are desired in particular recipient cells; whether it is desired for the vector to integrate into the chromosome or to remain extra-chromosomal in the recipient cells; and whether it is desirable to be able to "shuttle" the vector between recipient cells of different species.

Expression vectors can be autonomous or integrative. A recombinant nucleic acid can be in introduced into the host cell in the form of an expression vector such as a plasmid, phage, transposon, cosmid or virus particle. The recombinant nucleic acid can be maintained extrachromosomally or it can be integrated into the cell chromosomal DNA. Expression vectors can contain selection marker genes encoding proteins required for cell viability under selected conditions (e.g., URA3, which encodes an enzyme necessary for uracil biosynthesis or TRP1, which encodes an enzyme required for tryptophan biosynthesis) to permit detection and/or selection of those cells transformed with the desired nucleic acids. Expression vectors can also include an autonomous replication sequence (ARS).

Integrative vectors generally include a serially arranged sequence of at least a first insertable DNA fragment, a selectable marker gene, and a second insertable DNA fragment. The first and second insertable DNA fragments are each about 200 (e.g., about 250, about 300, about 350, about 400, about 450, about 500, or about 1000 or more) nucleotides in length and have nucleotide sequences which are homologous to portions of the genomic DNA of the host cell species to be transformed. A nucleotide sequence containing a gene of interest for expression is inserted in this vector between the first and second insertable DNA fragments, whether before or after the marker gene. Integrative vectors can be linearized prior to transformation to facilitate the integration of the nucleotide sequence of interest into the host cell genome.

A vector can be introduced into a host cell using a variety of methods. Methods of transfection foreign DNA into a host cell of are known in the art and can involve instruments (e.g. electroporation, biolistic technology, microinjection, laserfection, opto-injection) or reagents (e.g. lipids, calcium phosphate, cationic polymers, DEAE-dextran, activated dendrimers or magnetic beads), can be virus-mediated or by any other means known by the skilled person. In stable transfections, cells have integrated the foreign DNA in their genome. In transient transfections, the foreign DNA does not integrate in the genome but genes are expressed for a limited time (24-96h). The term "transformation" is used to describe foreign DNA transfer in bacteria and non-animal eukaryotic cells. This can be obtained by heat-shock of chemically competent bacteria, by electroporation or other methods of transformation known in the art.

The term "host cell" as used herein, refers to the cell that has been introduced with one or more polynucleotides, preferably DNA, by transfection. By means of an example, the host cell may be a bacterial cell, a fungal cell, including yeast cells, an animal cell, or a mammalian cell, including human cells and non-human mammalian cells. More particularly, expression systems or host cells that can be used for small or large scale production of polypeptides include, without limitation, microorganisms such as bacteria (e.g., *Escherichia. coli, Yersinia enterocolitica, Brucella* sp., *Salmonella tymphimurium, Serratia marcescens,* or *Bacillus subtilis*)*,* for example transformed with recombinant bacteriophage DNA, plasmid DNA, or cosmid DNA expression vectors; or fungal cells (e.g., *Yarrowia lipolytica, Arxula adeninivorans,* methylotrophic yeast (e.g., methylotrophic yeast of the genus *Candida, Hansenula, Oogataea, Pichia* or *Torulopsis,* e.g., *Pichia pastoris, Hansenula polymorpha, Ogataea minuta,* or *Pichia methanolica*), or filamentous fungi of the genus *Aspergillus, Trichoderma, Neurospora, Fusarium,* or *Chrysosporium,* e.g., *Aspergillus niger, Trichoderma reesei,* or yeast of the genus *Saccharomyces or Schizosaccharomyces,* e.g., *Saccharomyces cerevisiae,* or *Schizosaccharomyces pombe),* for example transformed with recombinant fungal expression vectors. Useful expression systems also include insect cell systems (e.g., cells derived from *Drosophila melanogaster,* such as Schneider 2 cells, cell lines derived from the army worm *Spodoptera frugiperda,* such as Sf9 and Sf21 cells, or cells derived from the cabbage looper *Trichoplusia ni,* such as High Five cells) infected with recombinant virus expression vectors (e.g., baculovirus) containing the nucleic acid molecules, and plant cell systems infected with recombinant virus expression vectors (e.g., tobacco mosaic virus) or transformed with recombinant plasmid expression vectors (e.g., Ti plasmid). Mammalian expression systems include human and non-human mammalian cells, such as rodent cells, primate cells, or human cells. Mammalian cells, such as human or non-human mammalian cells, may include primary cells, secondary, tertiary etc. cells, or may include immortalised cell lines, including clonal cell lines. Preferred animal cells can be readily maintained and transformed in tissue culture. Non-limiting example of human cells include the human HeLa (cervical cancer) cell line. Other human cell lines common in tissue culture practice include *inter alia* human embryonic kidney 293 cells (HEK cells), DU145

(prostate cancer), Lncap (prostate cancer), MCF-7 (breast cancer), MDA-MB-438 (breast cancer), PC3 (prostate cancer), T47D (breast cancer), THP-1 (acute myeloid leukemia), U87 (glioblastoma), SHSY5Y (neuroblastoma), or Saos-2 cells (bone cancer). A non-limiting example of primate cells are Vero (African green monkey Chlorocebus kidney epithelial cell line) cells, and COS cells. Non-limiting examples of rodent cells are rat GH3 (pituitary tumor), CHO (Chinese hamster ovary), PC12 (pheochromocytoma) cell lines, or mouse MC3T3 (embryonic calvarium) cell line. Such cells, prior to the genetic engineering as specified herein, can be obtained from a variety of commercial sources and research resource facilities, such as, for example, the American Type Culture Collection (Rockville, MD). Various promoters may be suitable for expression in mammalian cells, e.g., the metallothionein promoter, the adenovirus late promoter, or the cytomegalovirus promoter.

Bacteria may be all bacterial species as known by the one skilled in the art. Preferably, bacterial species that can be used in a biosafety level (BSL) 1 or 2 (BSLs for bacteria are determined by, for example, U.S. Public Health Service guidelines or in the Council Directive 90/679/EEC of 26 November 1990 on the protection of workers from risks related to exposure to biological agents at work, OJ No. L 374, p. 1.)

In particular embodiments, the host cell according to the invention is a bacterial cell, preferably *Escherichia Coli, Porphyromonas gingivalis, Capnocytophaga canimorsus, Capnocytophaga canis* or *Capnocytophaga cynodegmi, Flavobacterium johnsoniae,* more preferably *Capnocytophaga canimorsus.*

Another aspect of the invention is a method for producing a polypeptide having protease activity comprising (a) cultivating the host cell according to the invention under conditions conducive to expression of the polypeptide; and (b) recovering the polypeptide

The recovery of the peptide can be achieved by use of a tag. Affinity tags are appended to peptides so that they can be purified from their crude biological source using an affinity technique. Examples of such tags include chitin binding protein (CBP), maltose binding protein (MBP), glutathione-S-transferase (GST) and poly (His) tag.

The present invention is further illustrated in the following non-limiting examples.

### EXAMPLES

### Example 1: The N-term of FX LC and HC is cleaved by the C. canimorsus 5 protease C. canimorsus DPP7

The *C. canimorsus Dpp7* gene with a C-terminal Strep-His tag was inserted into the expression plasmid pKH32, which resembles pKH31, but encodes a Strep-His tag including a linker instead of the HA tag and cloned in *C. canimorsus 5.* A two-step purification protocol was carried out with His-tag affinity chromatography, followed by a Strep-tag affinity chromatography. The purity was confirmed by Silver staining (Figure 5 and 6). First, the activity of *C. canimorsus* DPP7 on NPP was tested. Incubation of NPP with the purified protocol led to prolonged PT and aPTT values (Figure 2A). A catalytic mutant of *C*. *canimorsus* DDP7 that was constructed by replacing the serine within the active site by an alanine was used as a control. This catalytic mutant did not have any impact on the clotting time. Incubation of purified FX with *C. canimorsus* DPP7 induced band shifts (Figure 2B) to a similar extent as was observed in plasma spiked with radioactive FX (data not shown). The band shift was absent if the incubation was performed with the catalytic mutant.

All factors affected by *C. canimorsus* 5 were VKD factors meaning they all have gamma-carboxy glutamic acids (Gla) at their N-terminus which allows interaction with calcium and mediates conformational changes of the proteins facilitating membrane interactions. Cleavage of this Gla domain could severely impact factor activity. By means of an antibody that specifically binds to an N-terminal epitope of the FX light chain that comprises amino acids 41-60 and includes 5 Gla residues, the Applicants investigated whether the *C*. *canimorsus* DPP7 exerted amino-terminal cleavage. Indeed, after incubation of FX with *C*. *canimorsus* DPP7, the antibody didn't bind anymore to the FX LC indicating that there was indeed N-terminal cleavage (Figure 2C).

There is no antibody binding the N-terminus of the heavy chain of FX available. Therefore, an N-terminal sequencing of FX was carried out. The results indicate the absence of 10 N-terminal amino acids of HC and one or two residues of LC. The N-terminal 53 AA of HC represent the activation peptide (AP), which is recognized and bound within the active site pocket of FVII or FIX and is then cleaved-off to generate FXa. It could thus well be that the *C*. *canimorsus* DPP7 induced cleavage of terminal amino acids at this place strongly reduces the activation of FX. It has indeed been demonstrated that removal of all but three amino acids from the AP of porcine FX resulted in a much slower activation rate by intrinsic Xase, the kcat for the activation of des-AP FX is 100-fold smaller than for FX (Duffy et al., Intrinsic pathway activation of factor X and its activation peptide-deficient derivative, factor Xdes-143-191. J Biol Chem. 1992 ; 267 :7821-7). The activation of human FX by the extrinsic Xase, on the contrary, seems not to require the activation peptide (Baugh et al., Role of the activation peptide domain in human factor X activation by the extrinsic Xase complex, J Biol Chem. 1996 ; 271 :16126-34, 1996).

### Example 2: The impact of C. canimorsus DPP7 on isolated FII, FVII, FIX and FX

Purified zymogens **FII**, FVII, FIX and FX were incubated with *C. canimorsus* DPP7 protease and added to the appropriate factor deficient plasma and immediately triggered coagulation by the addition of Neoplastin R (for **FII**, FVII and FX) or HemosIL reagent (for FIX). Incubation of FX with *C. canimorsus* DPP7 led to a 10-fold increase of the PT (Figure 3A). Incubation of FIX or FVII with *C. canimorsus* DPP7 caused almost a doubling of the aPTT or a 30% rise in the PT, respectively (Figure 3B and C). Interestingly, the effect of *C*. *canimorsus* DPP7 on FII was only very minor (Figure 3D). As a control, in order to exclude an effect of *C. canimorsus* DPP7 on the factor deficient plasma at the moment of the measurement, *C. canimorsus* DPP7 was directly added together with untreated factors to the plasma.

### Example 3: Purification of C. canimorsus DPP7 from E. coli BL21

The *C. canimorsus dppS* gene with a C-terminal Strep-His tag was inserted into the pET22b plasmid and cloned in *E. coli* BL21 (DE3).1 L of BL21 in LB were induced with IPTG 0.5µM for 4 hours at 30°C. Bacteria were pelleted and snap frozen. Lysis was performed in Tris-HCl containing NaCl and Imidazole with a French Press at 2.4 kbar. Purification was carried out with a His-tag affinity (Figure 4), followed by a Strep-tag affinity resin (Figure 5). Elution fraction (EF) 2 and 3 were centrifuged with a 30 kDa cut off filter and concentrated to a 2X solution. The final concentrations were 88.4 ug/ml for EF2 and 36 ug/ml for EF3. The activity of purified *C. canimorsus* DPP7 was determined in normal pooled plasma (NPP) (1:5 dilution) after 1 hour incubation at 37°C. The prothrombin time (PT) of EF2 at a concentration of (17.7 ug/ml) was 54s, while the PT of EF3 at a concentration of 7.2ug/ml was 21 s.

### Example 4: Purification of C. canimorsus DPP7 from C. canimorsus 5

The purification was carried out with Δ*dppS* (pKH32) and His-tag affinity (Figure 6), followed by a Strep-tag affinity resin (Figure 7). pKH32 encodes a C-terminal Strep-His tagged version of *C. canimorsus* DPP7 whose expression is controlled by the *ompA* promotor from *F*. *johnsoniae* to guarantee higher expression levels. The final product was tested for its activity by incubating several concentrations (50, 100 and 200 ng/ml) of *C. canimorsus* DPP7 in normal pooled plasma (NPP) during 1 hour at 37°C. Figure 2A shows that aPTT and PT decreased with increasing concentration of *C. canimorsus* DPP7.

## Claims

1. A DPP7 peptide, or functionally active variants or fragments thereof, for use in the cleavage and inactivation of coagulation factor X, VII and/or IX.

2. The DPP7 peptide, or functionally active variants or fragments thereof, for use according to claim 1, in the treatment and/or prevention of a medical disorder chosen from the list comprising thromboembolism caused by atrial fibrillation, pulmonary embolism, deep vein thrombosis, venous thromboembolism, congestive heart failure, stroke, coronary syndrome, peripheral arterial occlusion, myocardial infarction and/or genetic or acquired hypercoagulability.

3. *In vitro* use of a DPP7 peptide, or functionally active variants or fragments thereof, in the cleavage and inactivation of coagulation factor X, VII and/or IX.

4. *In vitro* use of the DPP7 peptide, or functionally active variants or fragments thereof, according to claim 3, for producing an anticoagulant reversal agent, wherein said anticoagulant is a factor X(a), VII(a) or IX(a) inhibitor.

5. *In vitro* use of the DPP7 peptide, or functionally active variants or fragments thereof, according to claim 3, for depleting a plasma sample of coagulation factor X, VII and/or IX.

6. *In vitro* use of the DPP7 peptide, or functionally active variants or fragments thereof, according to claim 3, in medical devices.

7. The DPP7 peptide, or functionally active variants or fragments thereof, for use according to claim 1 or 2, or the *in vitro* use of the DPP7 peptide, or functionally active variants or fragments thereof, according to claim 3 or 6, as an anticoagulant and, optionally wherein said DPP7 peptide is bacterial DPP7, preferably *Porphyromonas gingivalis* DPP7, *Capnocytophaga canimorsus* DPP7, *Capnocytophaga canis nov. sp.* DPP7 or *Capnocytophaga cynodegmi* DPP7, more preferably *Capnocytophaga canimorsus* DPP7.

8. The DPP7 peptide for use according any one of claims 1, 2 or 7, or the *in vitro* use of the DPP7 peptide according to any one of claims 3 to 7; wherein said peptide comprises an amino acid sequence which has at least 90%, preferably at least 95%, most preferably 100% identity with the amino acid sequence set forth in SEQ ID NO:1, or functionally active variants or fragments thereof.

9. The DPP7 peptide for use according any one of claims 1, 2, 7 or 8, or the *in vitro* use of the DPP7 peptide according to any one of claims 3 to 8; wherein said peptide comprises:
(a) an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(b) an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(c) an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1 ;
or functionally active variants or fragments thereof.

10. A kit comprising a DPP7 peptide, or functionally active variants or fragments thereof, wherein said peptide or functionally active variants or fragments thereof, has anticoagulant activity, preferably by inhibiting clotting factor X, VII and/or FIX, preferably wherein said DPP7 peptide comprises an amino acid sequence which has at least 90%, preferably at least 95%, most preferably 100% identity with the amino acid sequence set forth in SEQ ID NO:1, or functionally active variants or fragments thereof.

11. A kit comprising one or more plasma samples depleted of coagulation factor X, VII and/or IX, wherein said one or more plasma samples are depleted of coagulation factor X, VII and/or IX using a DPP7 peptide or functionally active variants or fragments thereof.

12. A nucleic acid construct comprising a nucleic acid sequence which
(a) has at least 90%, preferably at least 95%, most preferably 100% identity with the nucleic acid sequence set forth in SEQ ID NO:2, and/or encodes a peptide comprising:
(b) an amino acid sequence which has at least 90%, preferably at least 95%, most preferably 100% identity with the amino acid sequence set forth in SEQ ID NO: 3 or 6, comprising a histidine at an amino acid position corresponding to the position of amino acid 86 of the DPP7 polypeptide as set forth in SEQ ID NO: 1,
(c) an amino acid sequence which has at least 90%, preferably at least 95%, most preferably 100% identity with the amino acid sequence set forth in SEQ ID NO: 4, comprising an aspartic acid at an amino acid position corresponding to the position of amino acid 193 of the DPP7 polypeptide as set forth in SEQ ID NO: 1, and/or
(d) an amino acid sequence which has at least 90%, preferably at least 95%, most preferably 100% identity with the amino acid sequence set forth in SEQ ID NO: 5 or 7, comprising a serine at an amino acid position corresponding to the position of amino acid 649 of the DPP7 polypeptide as set forth in SEQ ID NO: 1 and a glycine at an amino acid position corresponding to the position of amino acid 667 of the DPP7 polypeptide as set forth in SEQ ID NO: 1,
operably linked to one or more control sequences capable of directing the expression of the polypeptide in a suitable expression host.

13. A recombinant expression vector comprising the nucleic acid construct of claim 12, a promoter, and transcriptional, translational stop signals, and preferably, a selectable marker.

14. A recombinant host cell comprising the nucleic acid construct of claim 12, preferably contained on a vector or integrated into the host cell genome; optionally, wherein the host cell is a bacterial cell, preferably *Escherichia Coli, Porphyromonas gingivalis, Capnocytophaga canimorsus, Capnocytophaga canis* or *Capnocytophaga cynodegmi, Flavobacterium johnsoniae,* more preferably *Capnocytophaga canimorsus.*

15. A method for producing a polypeptide having protease activity comprising (a) cultivating the host cell of claim 14 under conditions conducive to expression of the polypeptide; and (b) recovering the polypeptide.
